# EUROPEAN PATENT SPECIFICATION

(11) **EP 0 275 598 B1**
(45) Date of publication and mention of the grant of the patent: **07.01.1998**
(21) Application number: 87202566.3
(22) Date of filing: 16.12.1987
(51) Int. Cl.: C12N 15/09, C12N 15/24, C12N 1/13, C12N 1/15, C12N 1/19, C12N 1/21, C12N 5/10, C12P 21/02, C12P 21/00, C12N 1/20, C12N 1/16

(54) **Molecular cloning and expression of human IL-3**
Molekulare Klonierung und Expression von menschlichem IL-3
Clonage et expression moléculaire de IL-3 humain

(30) Priority: 16.12.1986 EP 86202285; 13.07.1987 EP 87201322
(43) Date of publication of application: 27.07.1988
(60) Divisional application: 96203517.6
(73) Proprietor: GIST-BROCADES N.V., NL-2611 XT Delft (NL)
(72) Inventor: Dorssers, Lambertus Christiaan Johannes, Dr. Ir., NL-6668 AN Randwijk (NL); Wagemaker, Gerard, Dr., NL-2564 GT Den Haag (NL); Vos, Yvonne Johanna, NL-2907 MB Capelle a/d Ijssel (NL); Van Leen, Robert Willem, NL-6546 TW Nijmegen (NL)
(74) Representative: Renes, Johan

(56) References cited:
- EP-A- 0 138 133
- WO-A-85/02863
- WO-A-88/00598
- CELL, vol. 47, 10 October 1986; YU-CHUNG YANG et al., pp. 3-10
- CHEMICAL ABSTRACTS, vol. 103, no. 23, 09 December 1985, Columbus, OH (US); T. YOKOTA et al., p. 187, AN 190859c
- GENE, vol. 58, no. 1, 1987, Elsevier Science Publishers B.V., Amsterdam (NL); L. DORSSERS et al., pp. 115-124
- CELL, vol. 46, no. 5, 1986; pp. 659-667
- Chemical Abstracts, vol. 106, no. 11, 16.03.87 page 148, abstr. no. 79502d, Columbus, Ohio, US.

## Description

### Field of the Invention

The present invention relates to cDNA encoding human interleukin-3 (hIL-3) and its use, inter alia, in the cloning and expression in various organisms, including microorganisms, in particular yeasts, bacteria and fungi, tissue culture cells and transgenic animals and plants.

### Background of the Invention

Hemopoiesis involves the active process of proliferation and differentation of pluripotent progenitor cells into all types of mature blood cells and some specialized tissue cells. Production of functional blood cells is regulated by specific proteins, the hemopoietic growth factors (HGFs). Some of the HGFs control maturation of a specific maturation lineage, whereas others stimulate proliferation and differentiation of progenitors along multiple pathways. Much of our knowledge of the hemopoietic differentiation process has been obtained from mouse studies in vitro and in vivo, using purified growth factors. The murine growth factor interleukin-3 (mIL-3), also termed Multi-CSF, mast cell growth factor, stem cell activating factor or several other designations, stimulates the proliferation of developmentally early, multipotent cells (CFU-S) as detected by the spleen colony assay, resulting in the production of progenitor cells along the erythroid, megacaryocyte, granulocyte/macrophage, osteoblast and several other lineages. Furthermore, mIL-3 has been implicated in replication of pluripotent stem cells, probably in synergism with other HGFs.

In recent years, several groups have succeeded in cloning mIL-3 cDNA. No results have been reported sofar of identifying homologous sequences in human DNA using mIL-3 DNA as a probe. Presumably, the human gene has diverged extensively from the mIL-3 gene or has lost its function during primate evolution. However, human leukocytes were found to produce a HGF(s) which can replace mIL-3 in supporting the proliferation of murine CFU-S. Thus, the existence of a human HGF was postulated, which shares biological properties with mIL-3 and therefore could be the human homolog. Yang, Y-C, et al, Cell (1986) 47:3-10, dated 10 October discloses cDNA encoding a protein having IL-3 like activity from gibbon T-cells, and retrieval of a genomic DNA which encodes the human counterpart. The sequence of a cDNA encoding human IL-3 can be deduced from the human gene sequence published by Yang et al. However, said article does neither disclose nor teach a method for isolation of a cDNA encoding human IL-3, nor was the production of hIL-3 achieved. This invention describes for the first time the isolation of a cDNA comprising the entire coding sequence for human IL-3.

Human IL-3 protein has never been prepared in purified form, nor have its characteristics, other than its activity in certain in vitro proliferation assays and deduced primary structure, been disclosed. The present invention permits the recovery of purified human IL-3, and identification of its characteristics through recombinant production from a cDNA clone.

### Summary of the Invention

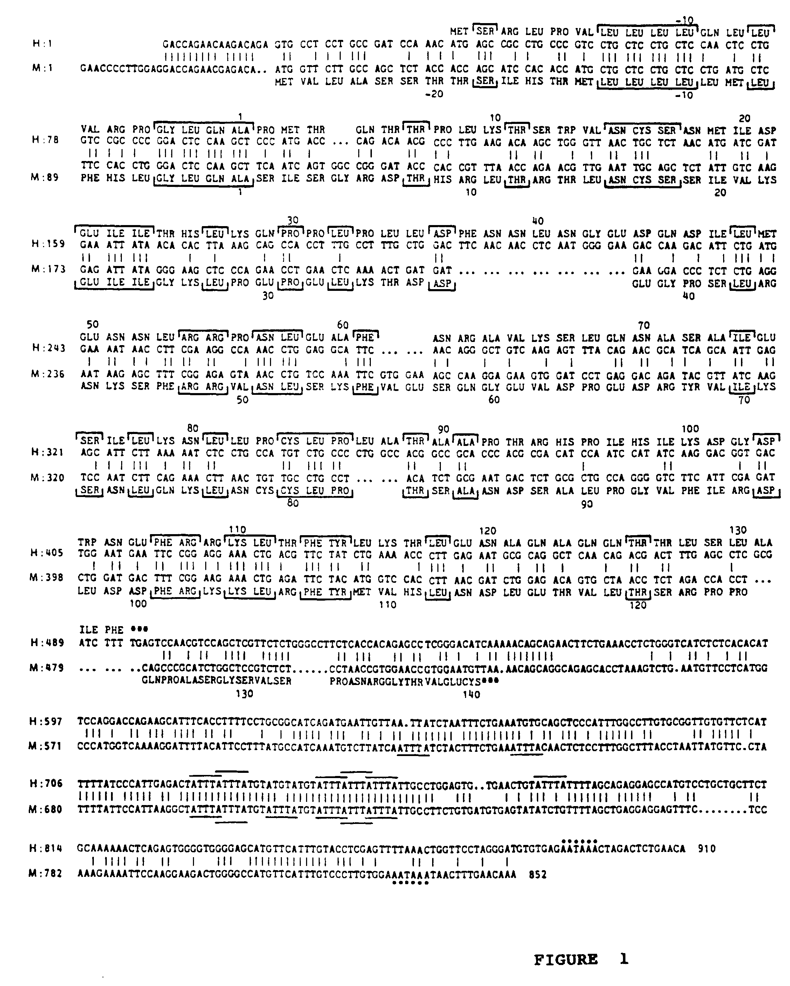

As stated above, the present invention for the time describes the isolation of a cDNA comprising the entire coding sequence for human IL-3 having a Pro at position 8 of the mature protein and having nucleotide sequence "H" of Figure 1. The low degree of homology between the DNA sequences coding for murine and human IL-3 does not permit the retrieval of a cDNA for hIL-3 by hybridization with the mIL-3 coding sequence. Unexpectedly, the hIL-3 cDNA clone could be isolated by exploiting the rather high degree of homology in the 3' noncoding part of the cDNA's. The availability of the cDNA clone permits the production of hIL-3 by a wide range of host organisms. Subsequent to large scale production the protein may be purified and used therapeutically.

The present invention permits production of recombinant human IL-3 having a Pro at position 8 of the mature protein and having nucleotide sequence "H" of Figure 1 in a wide range of host cells by transcription and translation from a cDNA sequence encoding the human IL-3 having a Pro at position 8 of the mature protein and having nucleotide sequence "H" of Figure 1. The production of the protein is illustrated in several hosts, including E. coli, COS cells, C127 cells, B. subtilis and B. licheniformis, S. cerevisiae and K. lactis, hereinbelow. Production in other hosts using appropriate expression systems is also made possible by provision of the intronless CDNA. More generally, the availability of antihuman IL-3 antibodies which permit identification of colonies exhibiting successful production of the recombinant protein aids in production of human IL-3 from any recombinant system.

In one aspect, therefore, the invention is directed to a recombinant, intronless, DNA encoding human IL-3 having a Pro at position 8 of the mature protein and having nucleotide sequence "H" of Figure 1. In another aspect, it is directed to expression systems capable of effecting the expression of said DNA sequence encoding hIL-3 having a Pro at position 8 of the mature protein and having nucleotide sequence "H" of Figure 1 in an appropriate host.

In other aspects, the invention is directed to recombinant human IL-3 having a Pro at position 8 of the mature protein and having nucleotide sequence "H" of Figure 1 in glycosylated or unglycosylated form, to human IL-3 free of substances normally accompanying said protein, and to antibodies specifically reactive with these recombinant or purified proteins.

### Brief Description of the Drawings

Figure 1 shows a comparison of DNA and protein sequences of human multi-CSF and mouse IL-3. The hmulti-CSF protein and DNA sequence (clone D11, top lines) were aligned with the mIL-3 DNA (11, 35) and protein sequence (30). Identical nucleotides are indicated by a vertical line, identical amino acids are shown in boxes. Black dots indicate a polyadenylation signal sequence and horizontal bars mark ATTTA repeat units.

Figure 2 shows the construction of plasmid pLB4 containing human IL-3 cDNA. E = EcoRI, Sm = SmaI, B = BamHI, S = SstI, K = KpnI.

Figure 3 shows the biological activity of COS/pLB4 CM on human bone marrow progenitors. The mean numbers of erythroid (BFU-E), granulocyte-macrophage (CFU-GM), granulocyte (CFU-G), eosinophil(CFU-Eo), macrophage (CFU-M) and mixed (CFU-MIX) colonies (±SD) are shown for duplicate cultures stimulated with graded volumes of COS/pLB4 CM.

Figure 4 shows induction of AML proliferation by COS/pLB4 CM as assessed in a colony culture assay (panel A) and in a DNA synthesis (³H-TdR incorporation) assay (panel B).

Figure 5 shows a construction diagram of the E. coli expression vectors pGB/IL-301, GB/IL-302, pGB/IL-303, pGB/IL-304, pGB/IL-305 and pGB/IL-306. In this Figure X stands for XhoI, E for EcoRI, B for BamHI and A for AvaI site.

Figure 6 shows the sequence of the multicloning site in pTZ18R (Pharmacia) and its derivative pT1.

Figure 7 shows a schematic presentation of hmulti-CSF expression clones. For the eucaryote expression plasmids pLB4 and pLH1 only the hmulti-CSF cDNA insert is shown. Leader peptide ( 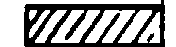 ) and mature hmulti-CSF protein ( 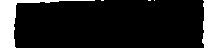 ) coding regions are indicated in boxes. Bacterial expression clones of hmulti-CSF (derived from pLH1) contain the lacZ and multi-linker protein coding region ( 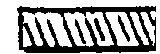 ), the 5' terminal noncoding region of hmulti-CSF ( 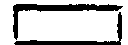 ) and the hmulti-CSF coding region. The arrow marks the ATG startcodon used in the particular vector.

Figure 8 shows the sequences of fusion regions of lacZ/hmulti-CSF DNA for various bacterial expression vectors. The sequence of clones is given from the start of the lacZ protein in either pUC8 or pTZ18R (lower case letters) and of hmulti-CSF DNA sequence up to the ClaI site at position 158. Mutations in the hmulti-CSF DNA sequence are underlined, resulting in: trp¹³→arg¹³ (pGB/IL-302); leu⁹→pro⁹ and trp¹³→arg¹³ (pGB/IL-303); met³→thr³ and a silent change (pGB/IL-304). The superscripts denote the amino acid residue number of the mature protein.

Figure 9 shows polyacrylamide gel-electrophoresis of bacterial hmulti-CSF produced from bacteria containing pGB/IL-301 and pGB/IL-302.

Figure 10 shows the titration of hmulti-CSF fusion protein on AML blast cells.

Figure 11 shows a Western blot demonstrating the IL-3 specific reaction of rabbit antisera raised against the 21 kDA protein isolated from a lysate of E. coli transformed with pGB/IL-301.

Figure 12 shows the effect of the antisera of Figure 11 on IL-3 activity.

Figure 13 shows a schematic representation of plasmid pGB/IL-307. The box ( 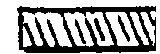 ) indicates the human IL-3 coding sequence. The N-terminal amino acids of the fusion protein are depicted below the drawing.

Figure 14 shows a schematic representation of plasmid pGB/IL-308. The nucleotide sequence of the promoter region is depicted below the drawing.

Figure 15 shows the construction of plasmid pGB/IL-309. The first box ( 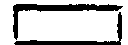 ) indicates a part of the human IL-3 sequence, viz. the signal sequence plus 20 amino acids of the mature protein. The other box ( 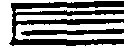 ) indicates part of the 3' noncoding region of the IL-3 cDNA sequence.

Figure 16 is a schematic representation of plasmid pGB/IL-310.

Figure 17 shows the nucleotide sequence of plasmid pBHAI.

Figure 18 shows the construction of the plasmids pGB/IL-311 and pGB/IL-312. The box ( 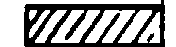 ) indicates the precursor human IL-3 coding region.

Figure 19 shows the construction of the plasmid pGB/IL-313. The sequence at the 5' side of the IL-3 sequence is depicted below the drawings.

Figure 20 shows a schematic representation of plasmid pGB/IL-317.

Figure 21 shows a schematic representation of plasmid pGB/IL-316.

Figure 22 shows the nucleotide sequence of plasmid pGB/IL-316 between the unique SacII site in the lactase promoter and the HindIII site behind the terminator (residues 4457 to 7204).

Figure 23 shows the nucleotide sequence of plasmid pGB/IL-318 between the unique SacII site in the lactase promoter and the HindIII site behind the terminator (residues 4457 to 7190).

Figure 24 shows the nucleotide sequence of the EF-1α promoter, SalI-BglII-XhoI linker and actin terminator as present on plasmid pGB/TEFact.

### Detailed Description of the Invention

### A. Definitions

As used herein, "human IL-3", "hIL-3", "human multi-CSF", and "hmulti-CSF" are used interchangeably, and designate a protein preparation which exhibits the following activities:
1. The protein stimulates colony formation by human hemopoietic progenitor cells wherein the colonies formed include erythroids, granulocytes, granulocyte macrophages, and mixed.
2. The protein stimulates DNA synthesis by human acute myelogenous leukemia (AML) blasts, as evidenced, for example, by labelled thymidine uptake.

To fit the definition of hmulti-CSF, the activity in the foregoing assay must not be substantially inhibited by antibodies raised in response to, and immunospecific for, GM-CSF, unless these antibodies also inhibit these activities by the illustrative hmulti-CSF below.

One illustrative form of hmulti-CSF is shown in Figure 1 as a 133 amino acid mature protein, having a 19 amino acid signal sequence. The amino acid sequence of Figure 1 is identical with that disclosed by Yang, Y-C., et al., Cell (1986) 47:3-10 (supra) except at position 8 of the mature protein wherein the Ser of the Yang protein is replaced by Pro herein. As shown herein, this amino acid sequence is effective in its nonglycosylated form. However, it contains two glycosylation sites, and the glycosylated form is also included within the scope of the invention. It is also recognized that the protein may exist in acid addition salt form, basic salt form, or may be neutral, depending upon the pH of its surroundings. Derivatization by phosphorylation, acetylation, and so forth, to the extent that activity is not destroyed, also results in a protein included within the scope of the invention.

It is also recognized that the entire sequence may not be necessary for activity. Parts of the amino acid sequence may be deleted or replaced, while retaining biological activity. As illustrated herein, the alanine at position 1 may be deleted, as may as many as the first fourteen amino acid residues if replaced by a sequence of residues of a fused peptide sequence. In addition, it is believed that the murine form of the protein requires only the first 79 residues for activity; this corresponds approximately to the first 83 residues of the human counterpart. Accordingly, fragments which comprise only the first 83 amino acid residues of the protein, and the N-terminal replaced forms thereof are also included within the scope of the invention. Furthermore, it should be considered that the N-terminus of mature hIL-3 is formed by the residues ala-pro-met etc. (see Figure 1). It is known that the protein, when secreted by a yeast host, may in some instances be shortened by two amino acids (ala-pro), due to the interaction with a dipeptidylaminopeptidase (72). The hIL-3 without the N-terminal alanine and proline still retains its biological activity. Yeast strains carrying a null mutation of the X-prolyl dipeptidylaminopeptidase gene will produce complete hIL-3 (amino acids 1-133). Accordingly, included in the multi-CSFs of the invention are those which contain and those which do not contain the N-terminal alanine and proline, produced by X-prolyl dipeptidylaminopeptidase mutants and wild type hosts, respectively.

When produced as a mature protein in a procaryotic host, the coding sequence for the mature protein will be prefaced by an ATG start codon. The resulting N-terminal methionine may then be removed, or partially removed, by processing within the bacterial host, depending on the nature of the subsequent amino acid sequence. Again, both forms of hIL-3 are biologically active. Therefore, included in the hmulti-CSFs of the invention are those which contain and those which do not contain the N-terminal methionine.

From the above it is clear that amino acid changes may be introduced into the human IL-3 protein, without affecting its biological function. It is recognized that minor changes in amino acid sequences by chemical modification of the encoded residue, substitution of a different residue, or deletion or addition of one or more, but preferably only one, residue results in proteins which retain activity. Accordingly, these nondestructive mutations are also included within the invention, in particular, the naturally occurring allelic variations and other mutations which are nonlethal to the activity.

On the other hand it should be considered that amino acid changes in the human IL-3 protein may be beneficial to the therapeutic use of the protein. As recognized herein, the mature protein has four conserved domains at residues 15-36, 54-61, 74-91, and 107-118. Proteins containing single and multiple amino acid changes in the nonconserved regions, 1-14 (which are, in any event, replaceable by the sequences of host derived fusion proteins), 37-53, 62-73, 92-106, and 119-133 are possible. However, it appears that the cysteine residues at positions 16 and 84 may be necessary for disulfide bridge formation as they are conserved between species. Changes in the conserved domains mentioned above may influence biological properties of the protein, such as receptor binding and signal transduction. It is envisaged that hIL-3 having altered properties are of therapeutic use. Such derivatives of hIL-3, which may be made by known protein engineering techniques, are to be understood within the scope of the present invention.

The protein preparation may contain the hmulti-CSF peptides in monomeric or aggregated form, provided the aggregates retain activity as above-defined.

As used herein, "expression system" refers to a DNA sequence which contains both a coding sequence whose expression is desired and appropriate control sequences in operable linkage with it which permits its expression when the control sequences are compatible with the host into which the expression system is placed. As is generally understood, "control sequences" refers to DNA segments which are required for or regulate the expression of the coding sequence with which they are operably linked.

Control sequences for all hosts include promoters, which may or may not be controllable by regulation of their environment. Typical promoters suitable for procaryotes include, for example, the trp promoter (inducible by tryptophan deprivation), the lac promoter (inducible with the galactose analog IPTG), the beta-lactamase promoter, and the phage-derived P_{L} promoter (inducible by temperature variation). Additionally, especially for expression in Bacillus, useful promoters include those for alpha-amylase, protease, Spo2 and synthetic promoter sequences. Suitable promoters for expression in yeast include the 3-phosphoglycerate kinase promoter and those for other glycolytic enzymes, as well as promoter regions for alcohol dehydrogenase and yeast phosphatase. Also useful are the transcription elongation factor (TEF) and lactase promoters. Mammalian expression generally employs promoters derived from viruses such as the adenovirus promoters and the SV40 promoter systems, but they also include regulatable promoters such as the metallothionein promoter, which is controlled by heavy metals or glucocorticoid concentration. There are also now available viral-based insect cell expression systems, as well as expression systems based on plant cell promoters such as the nopaline synthetase promoters.

In addition to the promoter DNA sequence, which is necessary for the transcription of the gene by RNA polymerase, a variety of control sequences, including those regulating termination (for example, resulting in polyadenylation sequences in eucaryotic systems) are also useful in controlling expression. Some systems also contain enhancer elements which are desirable but not necessarily necessary in effecting expression.

Translation controls include a ribosome binding site (RBS) in procaryotic systems, whereas in eucaryotic systems translation may be controlled by the nucleotide sequence around the AUG codon.

As implied above, recombinant protein production can be effected in a wide variety of hosts, including bacteria (predominantly E. coli, Bacillus, and Streptomyces), in yeast and fungi (such as Saccharomyces, Kluyveromyces, and Aspergillus), and in mammalian and other cell cultures such as COS cells, C127 cells, Chinese hamster ovary cells, Spodoptera frugiperda (Sf9) cells, and so forth. The protein may be produced as an intracellular mature or fusion protein, or may be secreted when the DNA encoding an appropriate compatible signal is included in the gene.

The present invention for the first time enables large scale production of recombinant human IL-3, so that this protein - in purified form - can now be used as a therapeutic agent. The methods described herein provide means for producing glycosylated as well as unglycosylated forms of the protein, which can be purified to substantially pure human IL-3. "Purified" human IL-3 refers to human IL-3 as defined above which is free of other proteins which normally accompany it.

### B. Retrieval of cDNA Encoding Human IL-3 having a Pro at position 8 of the mature protein and having nucleotide sequence "H" of Figure 1.

Human IL-3 having a Pro at position 8 of the mature protein and having nucleotide sequence "H" of Figure 1 was isolated according to the following strategy:
1. A procedure was developed which allowed for reproducible production if hemopoietic growth factors (HGFs) by human leucocytes.
2. mRNA was prepared from such producing cells and transcribed into double-stranded cDNA.
3. The cDNA was screened with a complete mIL-3 cDNA which contained both the coding and untranslated 3' downstream portions to obtain DII.
4. The hybridizing cDNA clone DII was inserted into an expression vector pLO to obtain pLB4 which was expressed in COS cells to confirm the presence of the sequence encoding human IL-3. Conditioned media from these cells showed the biological activity expected of hIL-3.

The human cDNA was retrievable using this procedure because despite considerable lack of homology with the murine coding sequence, a surprising degree of homology was present in the 3' untranslated region. Applicants are unaware of any prior disclosure of the use of a 3' untranslated sequence homology to retrieve an alternate species gene.

In more detail, conditioned medium of lymphocytes cultured in the presence of 12-O-tetradecanoylphorbol-13 acetate (TPA) and concanavalin A (Con A) is a suitable source for human HGFs as determined by assay of the medium using stimulation of mouse CFU-S is suspension cultures, proliferation of mIL-3 dependent DA-1 cells, human hemopoietic progenitor assays by colony formation in vitro, and in vitro stimulation of acute leukemia blasts. A cDNA library from human lymphocytes was constructed in lambda gt-10 phage (20) and screened using the HindIII-XbaI fragment of mIL-3 cDNA, for the occurrence of mIL-3 related sequences. No hybridizing clones were identified.

However, when complete murine IL-3 cDNA was used as probe, four clones were identified. Restriction enzyme analysis of the largest clone (D11) indicated a 910 bp insert containing an internal EcoRI site (at position 411, Figure 1).

(It was investigated whether this EcoRI site had arisen by ligation of two independent cDNA fragments or was a naturally occurring site. Southern analysis of restriction enzyme digested human DNA using labeled 5' and 3' EcoRI fragments of clone D11 as probe, revealed identical DNA fragments following digestion with HindIII (15 kb) and BamHI (4.6 kb). Furthermore, the DNA sequence around the EcoRI site does not correspond to linker sequence (pCCGAATTCGG) used for inserting cDNA into phage DNA, indicating that these EcoRI fragments are derived from a single mRNA.)

From hybridization and sequencing experiments it was concluded that the small clones (II, IV and VI) are identical to the 3' nucleotide sequence of clone D11 and derived from the same mRNA species.

Computer assisted alignment (Figure 1) of the D11 cDNA and the mIL-3 cDNA revealed sequence homology in the 5' terminal 100 bp, between nucleotides 236-269 and between nucleotides 598-803 in the 3' terminal region (68%, 71% and 73% homology, respectively). In particular, the region between nucleotides 706 and 763 is highly conserved (93% homology) and contains repetitive AT-rich sequences. The low homology in the 5' terminal 600bp of the human cDNA (52%) precludes detection by hybridization with the HindIII-XbaI fragment of mIL-3.

Analysis of the human cDNA clone for an encoded protein shows an open reading frame up to the termination codon TGA at position 495-497 (Figure 1). The first ATG triplet is probably the actual initiation codon of the encoded polypeptide. The putative encoded protein consists of a hydrophobic leader peptide of 19 amino acids, which is probably cleaved between the glycine and alanine residues (22, 23).

The alignment of the predicted amino acid residues of the human and mouse IL-3 (Figure 1) reveals a homology of 50% for the leader peptide (residues -26 to +1) and 28% for the mature protein (residues 1 to 133). Within the leader peptide, there are two conserved regions of four amino acids (residues -13 to -10 and -3 to +1), of which the second one encloses the processing site. The mature protein is 133 amino acids long and has a molecular weight of 15 kd. The mature protein has four conserved domains (residues 15-36, 54-61, 74-91 and 107-118) and contains two potential glycosylation sites (residues 15-17 and 70-72). Both cysteine residues present in the human protein (positions 16 and 84) are conserved and may play an essential role in protein folding by disulfide bridge formation.

In order to verify that this human cDNA encodes a functional protein that resembles mIL-3, the Dll cDNA was inserted in an eucaryotic expression vector (pLO, containing an SV40 transcription unit) to obtain the expression vector pLB4 and transfected to COS 1 cells. The COS/pLB4 conditioned medium (CM) was tested for (1) its capacity to stimulate colony formation by human bone marrow cells, and (2) to stimulate human acute myelogenous leukemia (AML) blasts.

In vitro colony growth of human hemopoietic progenitors depleted of myelomonocytic (Vim-2 positive) and T-lymphocytic (T-3 positive) accessory cells, was efficiently stimulated by COS/pLB4 CM. The data demonstrate stimulation of progenitors of several hemopoietic differentiation lineages and of a subpopulation of BFU-E by COS/pLB4 CM.

In a separate experiment, bone marrow was enriched for progenitor cells by density centrifugation, E-rosette sedimentation to remove T-lymphocytes and adherence to remove mononuclear phagocytes and cultured in enriched medium containing fetal calf serum. Under these conditions, the majority of the colonies obtained upon stimulation with COS/pLB4 CM contained two or more hemopoietic differentiation lineages: all contained macrophages, approximately half immature blasts and/or immature erythroid cells and/or neutrophilic granulocytes and a minority, in addition, basophilic or eosinophilic granulocytes. These results demonstrate the multilineage stimulatory properties of the protein encoded by the human cDNA clone D11 and its action on developmentally early, multipotent hemopoietic cells.

With respect to AML stimulation, AML blasts of five patients were stimulated with the COS/pLB4 CM and assayed for a response by measuring ³H-TdR incorporation and colony formation. Three of the five leukemia cell samples responded to the COS/pLB4 CM in both assays; characteristic dose-response relationships for colony formation and DNA synthesis of AML blasts of different patients were obtained. The responses to GM-CSF demonstrated further phenotypic differences among the leukemias responding to the COS/pLB4 CM.

These data demonstrate that the D11 cDNA clone contains the complete genetic information for a biologically active protein which is exported into the culture medium in the transformed COS cells. Despite the apparent lack of homology with respect to the protein sequence between the human protein and mIL-3 (only 30%), the proteins are comparable with respect to their biological function. Both proteins exert their effect on developmentally early hemopoietic progenitors of various lineages. The low homology at the amino acid level is also reflected by a low homology in the coding nucleotide sequence. However, very unexpectedly, a rather high degree of homology -- sufficient for retrieval of the human cDNA clone -- occurred in the 3' untranslated region.

Southern analysis of human DNA revealed a single hybridizing gene indicating that this cDNA does not belong to a family of closely related genes.

From the foregoing results we conclude that the human cDNA insert in D11 encodes the human homolog of mIL-3. We decided to use the operational term hmulti-CSF for the protein encoded by the cDNA clone Dll in view of its major biological effect and assay.

The identification of hmulti-CSF cDNA clones by virtue of hybridization with the 3' terminal region of the mIL-3 cDNA was unexpected. Whereas homologous DNA sequences are in general predominantly found in the coding region, the hmulti-CSF sequence has extensively diverged (45% homology) in this part of the gene. Analysis of the highly conserved domain in the 3' terminal non-coding region reveals the occurence of 5 ATTTA repeat units which are all preserved in the mIL-3 cDNA (Figure 1).

hMulti-CSF and mIL-3 display considerably less protein homology than other murine and human growth factors or lymphokines such as GM-CSF (25), interleukin-2 (25), interleukin-1 (26) and interferons (27-29). The biological activity of the mature mIL-3 appears to be contained in the first 79 amino acids, including an absolute requirement for the cysteine residue at position 17 (30). This cysteine residue is conserved in hmulti-CSF (Fig. 1, pos. 16) and may play an essential role in protein folding. The occurrence of a potential glycosylation site around this cysteine residue may interfere with disulfide bridge formation.

### C. Production and Formulation of hmulti-CSF

Applicants have provided a representative variety of expression systems capable of producing human IL-3 protein in a variety of forms -- as fusion proteins, as mature intracellular proteins, and as secreted proteins. Applicants are unware of availability anywhere in the art of recombinant forms of human IL-3, or, indeed, of any human IL-3 in a preparation which is free of proteins normally accompanying this desired protein. Accordingly, the invention herein provides, for the first time, the human IL-3 protein in a manner which is capable of adaptation to therapeutic and diagnostic uses.

The human IL-3 can be produced as a fusion protein with sequences heterologous to the human IL-3 amino acid sequence. By "heterologous" is meant a sequence which is not found in human IL-3 itself, but is an unrelated sequence. This heterologous sequence may be derived from a bacterial protein, a yeast protein, a mammalian protein, or any of variety of miscellaneous fortuitously encoded sequences such as, for example, those encoded by polylinkers. It is clear from the results hereinbelow that at least the first 14 amino acids of the N-terminus of the human IL-3 sequence can be replaced by a heterologous sequence, at least if the fusion protein is further extended past the N-terminus.

The protein can also be obtained as a mature intracellular protein by constructs in which the ATG start codon is placed immediately upstream of the desired N-terminus. These intracellular proteins, whether mature or fusion proteins, can be recovered by lysing the cells and purifying the human IL-3 using standard protein purification techniques.

Protein purification is simplified if the human IL-3 is secreted into the medium. When produced in mammalian cells with which the native signal sequence is compatible, this native signal sequence can be used to effect secretion into the medium. In bacterial or yeast systems, signal sequences compatible with these hosts, such as the penicillinase or alpha-amylase sequence in bacteria or the alpha-factor signal sequence in yeast can be used.

When produced recombinantly, the human IL-3 is free of proteins normally accompanying it, and can be purified from the proteins and other materials indigenous to the recombinant host using, for example, chromatographic methods, gel filtration, ammonium sulfate precipitation, and so forth.

As described hereinbelow, the protein is useful for therapeutic and diagnostic purposes. For therapeutic uses, the protein may be formulated in ways standard for pharmaceutical compositions which are used for the administration of proteins. Suitable excipients include, for example, physiological saline, Ringer's solution, and so forth. Alternate formulations, including solid formulations (e.g. lyophilized), can also be employed.

### D. Preparation of Antibodies

The availability of recombinant IL-3 protein or parts thereof will permit production of antibodies directed against the protein or parts thereof, as demonstrated hereinbelow. Such antibodies are useful, inter alia, for in vitro detection of colonies producing hIL-3, for therapeutical use, and for the purification of both natural and recombinant hIL-3.

### Statement of Utility

The nucleotide sequence of the whole or parts of the cDNA of human IL-3, or closely-related DNA sequences will advantageously enable the detection of genetic abnormalities, including genomic rearrangements, restriction fragment-length polymorphisms, mutations and altered gene expression with the use of such techniques as the analysis of chromosomal DNA using restriction enzymes, DNA and RNA blotting as well as hybridization techniques (Maniatis et al. 1982) and two-dimensional gel electrophoresis (Fisher and Lerman, 1983).

The recombinant hmulti-CSF as provided by the present invention will facilitate a detailed analysis of its role in human hemopoiesis, in particular the possible synergism of hmulti-CSF and various other HGFs. Furthermore, hmulti-CSF is of considerable interest because of its applicability for in vitro diagnosis of human diseases in which hemopoietic progenitor cells are involved, which include the leukemia, as well as potential therapeutic applications aimed at expansion of hemopoiesis in vivo. The effect of hmulti-CSF on various hemopoietic malignancies with respect to terminal differentiation of the leukemic cells also needs to be explored. In addition hMulti-CSF may be required for establishing a proliferative state of human stem cells in gene therapy protocols, since stimulation with mIL-3 was shown to be required for successful infection of mouse stem cells with recombinant, replication defective retroviruses.

IL-3 protein can also advantageously be used for the detection of early hemopoietic precursor cells in standardised in vitro cultures (Wagemaker and Visser, 1980; Metcalf et al. 1982; Merchav and Wagemaker, 1984, Metcalf, 1986).

IL-3 protein and variants can further be used for the multiplication of hemopoietic stem cells in vitro, possibly in conjunction with other growth factors, for bone marrow transplantation and the genetic manipulation of stem cells (Lowenberg and Dicke, 1977; Wagemaker and Petem, 1978; Lemischka et al, 1986).

The IL-3 protein can be used for the determination of the response pattern of malignant hemopoietic cells in in vitro tests (Touw and Lowenberg, 1985; Griffin et al, 1986; Griffin and Lowenberg, 1986).

The IL-3 protein can further be used for the detection of remaining leukemic cells by in vitro methods (Touw and Lowenberg, 1986; Griffin et al, 1986; Griffin and Lowenberg, 1986).

Furthermore, the IL-3 protein can be used in vivo for the treatment and prevention of malignant and non-malignant disorders, either by itself or in combination, in which an obtained specific response by the hemopoietic system can result in a clinical benefit.

These applications include:
- cytopenias and/or immunosuppression due to infections such as AIDS
- cytopenias due to chemotherapy and/or irradiation
- bone disorders such as bone fractures and osteoporosis
- immunodeficiencies due to general anaesthetic procedures
- recovery following bone marrow transplantation
- adjunct to vaccinations and adjunctive therapy of infections.

The cloned human IL-3 DNA sequence or closely-related DNA can be used for gene therapy in genetic deviations from the normal IL-3 gene.

To facilitate the above-described analysis, a large quantity of human IL-3 is required. The easiest way to obtain sufficient amounts of the protein is the production with microorganisms, in particular yeasts, bacteria and fungi, e.g. Saccharomyces, Kluyveromyces, Aspergillus, Streptomyces, Bacillus and E. coli species. Production in mammalian and other eucaryotic systems, such as C127 cells, Spodoptera cells and transgenic animals and plants, is also possible for skilled persons following the teaching of the present invention. These possibilities are all included within the scope of this invention.

As an illustration how to obtain living cells that produce the human IL-3 protein by expression of the hIL-3 cDNA, a number of plasmids were constructed and transferred to E. coli, B. subtilis, B. licheniformis, S. cerevisiae, K. lactis and C127 cells. Using these host strains the production of recombinant human IL-3 was achieved. The products were tested for their capacity to stimulate human AML blasts as described above for the COS/pLB4 conditioned medium. From these experiments it appeared that the proteins made were biologically active.

The following examples are intended to illustrate but not to limit the invention.

### Example 1

### Retrieval of cDNA Encoding Human multi-CSF (hmulti-CSF)

Human leukocytes stimulated with TPA (5 ng/ml) and ConA (10 ug/ml) produced considerable amounts of HGFs as measured by the murine stem cell proliferation assay and various other colony assays. Cells were harvested 24 hrs after stimulation, because mRNA production is often transient following stimulation with phorbol esters and lectins. Already after 24 hrs, HGFs were easily detectable in the CM.

### mRNA Preparation

Cells were harvested, washed with PBS and homogenized in guanidinium isothiocyanate solution (36). RNA was pelleted through a cesium chloride cushion. Oligo(dT)-cellulose chromatography was used for selection of mRNAs (36).

### cDNA Synthesis

cDNA was synthesized essentially according to Gubler and Hoffman (37), using oligo(dT) as primer and AMV reverse transcriptase. Second strand was synthesized with RNaseH and E. coli DNA polymerase I. Gaps were closed with T4-DNA ligase and ends were flushed by T4-DNA polymerase. To protect internal EcoRI restriction sites, the cDNA was methylated with EcoRI methylase. Subsequently, the cDNA was ligated to phosphorylated EcoRI linkers with T4-DNA ligase. After digestion with EcoRI, excess linkers were removed by Sepharose CL-4B chromatography. The material recovered in the void volume of the column was larger than 250bp and was used for construction of the libraries.

### Construction of the Phage cDNA Library.

The cDNA was ligated to lambda gt10 phage arms (20) and packaged with commercial packaging extracts (Gigapack, Vector Cloning Systems). The recombinant phages were propagated in E. coli C600 hfl.

### Screening of the Phage Library.

Of each plate containing 1-5000 plaques, two nitrocellulose filter replicas were made according to standard procedures. Filters were then hybridized with radiolabeled mIL-3 probe from the HindIII-XbaI fragment of mIL-3 cDNA or with the complete mIL-3 cDNA clone radiolabeled with random primers. The mIL-3 cDNA clone (pL101) was isolated from a WEHI-3B cDNA library. WEHI-3B mRNA was isolated using the guanidinium isothiocyanate CsCl method, size fractionated on sucrose gradient and injected into Xenopus laevis oocytes. RNA fractions inducing the oocytes to produce a factor capable of supporting murine stem cell proliferation, were used for synthesis of cDNA as described above, cDNA was tailed with dC residues and inserted in the PstI site of pUC9. mIL-3 clones were identified using synthetic oligonucleotides (from published mIL-3 sequence, 11). Insert of pL101 was purified on polyacrylamide gel and used for screening of the human cDNA library. Probe DNA was labeled using the random primer method (38). Potential positive plaques were rescreened and plaque purified. In this way four clones were identified, including phage D11.

### Sequencing of cDNA Clones.

Recombinant phages were grown at large scale and purified, cDNA inserts were removed from the phage arms by digestion with EcoRI and purified on polyacrylamide gel. The purified fragments were ligated into M13mpl8 and pTZ18R DNA digested with EcoRI and used for transformation of E. coli JM109. Single strand DNA was prepared and sequenced according to established procedures (39). Sequence data were analyzed using various computer programs (40-43).

The sequence obtained for the insert in phage D11 is shown in Figure 1. This 910 bp sequence contains the entire coding region for hmulti-CSF and its signal sequence, and exhibits high homology to the murine clone pL101 in the 3' untranslated region. The homology upstream in the coding sequence is relatively more limited. AS described above, the protein has a putative 19 amino acid signal sequence followed by a 133 amino acid mature protein containing two glycosylation sites (15-17 and 70-72) and two cysteine residues at 16 and 84.

The deduced amino acid sequence is the same as that encoded by the genomic DNA disclosed by Yang, Y-C, et al. (supra), except for one amino acid -- that at position 8 of the putative mature protein; the Yang DNA encodes Ser, the cLNA herein encodes Pro.

The intronless sequence obtained in the phage D11 can be used for procaryotic expression, as well as for expression in eucaryotic systems, as illustrated below.

### Example 2

### Expression in Mammalian Cells

### A. Construction of the eucaryote expression vector pLB4

Phage D11 (containing the longest cDNA insert) was digested with HindIII and BglII and subcloned in plasmid pTl (a derivative of pTZ18R, containing some additional restriction sites in the multilinker, see Example 3A). Clones containing the phage fragment containing the cDNA insert were identified by restriction analysis. The cDNA insert was removed from this plasmid by partial digestion with EcoRI and purified by polyacrylamide gel electrophoresis. The appropriate fragment was inserted in a eucaryote expression vector (pLO) in an SV40 transcription unit.

pLO comprises: EcoRI (filled in) - PstI of pBR322 (1-755), PstI-AvaI of pBR329 (756-1849), AvaI-PvuII adapter (1850-1868), PvuII-HindIII (filled in) of SV40 (promoter) (1869-2211), PvuII-BamHI adapter containing the unique EcoRI site (2211-2251), MboI "splice fragment" of SV40 (2252-2861), BclI-BamHI (filled in) "poly A fragment" of SV40 (2862-3098), PvuII-HindIII promoter fragment of SV40 (3099-3440), HindIII-BamHI Eco gpt gene (3441-4501), MboI "splice fragment" of SV40 (4502-5111) and the BclI-BamHI (filled in) "poly A fragment" of SV40 (5112-5348).

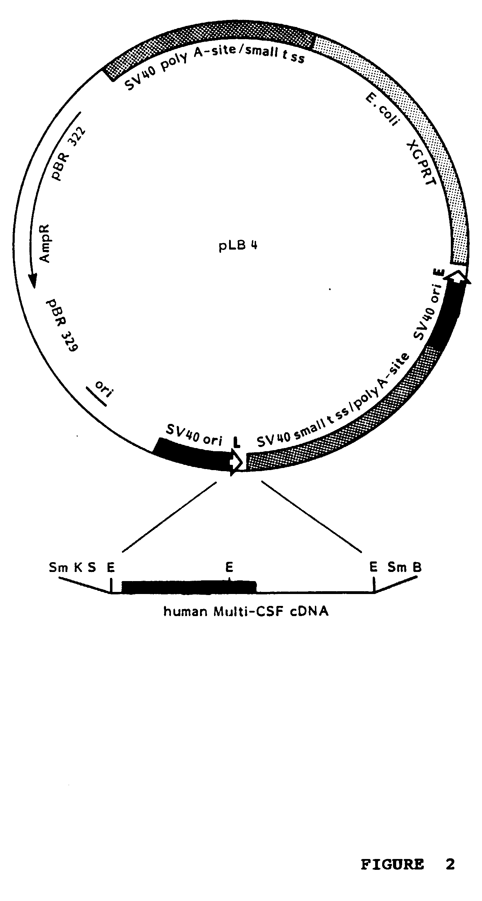

The Eco gpt transcription unit is of no importance in transient expression of proteins in COS 1 cells. The resultant expression plasmid for hmulti-CSF was termed pLB4 and was purified on CsCl. This plasmid in E. coli was deposited with the Centraal Bureau of Schimmelcultures (CBS), Baarn, the Netherlands, under the provisions of the Budapest Treaty on December 12, 1986 under CBS 568.86. The construct is shown in Figure 2.

### B. Expression of hmulti-CSF in COS 1 Cells and Bioassays.

pLB4 DNA was transfected to COS 1 cells using the calcium phosphate coprecipitation method (45). Cells were cultured for 48-72 hours in alpha medium containing 10% fetal calf serum. The culture medium was recovered, filtered and used in assays for establishing its biologic activity. Human bone marrow progenitor colony assays and acute myeloid blasts colony and proliferation assays were performed as follows. Bone marrow was obtained from hematologically normal adult volunteers by posterior iliac crest puncture following informed consent. The mononucleated cells were separated by density gradient centrifugation on a Ficoll gradient (Nijegaard and Co., Oslo, Norway), washed and resuspended in Hanks balanced salt solution (HBSS). Myeloid cells and T-lymphocytes were then removed. For this purpose, marrow cells were lysed following incubation with monoclonal antibodies OKT-3 (CD3; Ortho, Ravitan, N.Y.) and Vim 2 (myelo-monocytic cells, 46) at saturating concentrations in the presence of rabbit complement (40%; 30 minutes, 25°C) according to established procedures (47). The cells were washed two times in HBSS, resuspended in Iscove's modified Dulbecco's medium (IMDM) and cultured in the presence of autologous plasma according to Fauser and Messner (16), as described before (48), at a concentration of 1.5-3 x 10⁴/ml. Erythropoietin 1 U/ml (sheep, step III, Connaught, Willowdale, Canada) and COS/pLB4 CM were added as growth stimulating activities. Results of standard cultures with phytohaemagglutinin stimulated leukocytes CM (PH-LCM) in direct comparison with COS/pLB4 CM are also given. Sixty percent of the colonies were plucked and identified by microscopical analysis. The CM from COS cells transfected with the vector without insert (pLO) failed to stimulate colony formation by itself.

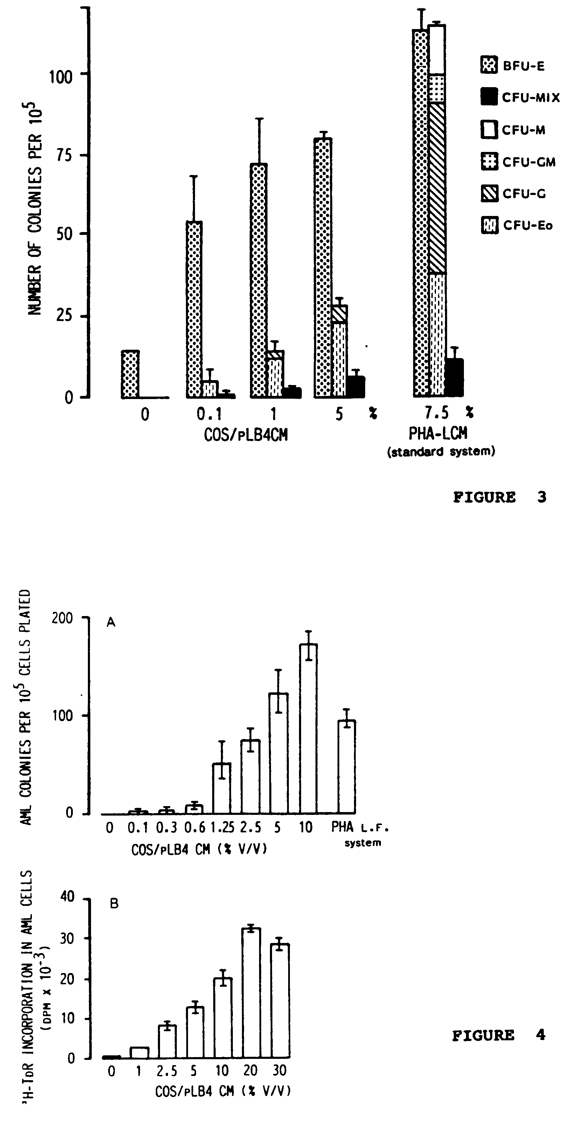

The results are shown in Figure 3. As shown in the figure, the mean numbers of erythroid (BFU-E), granulocyte-macrophage (CFU-GM), granulocyte (CFU-G), eosinophil (CFU-Eo), macrophage (CFU-M) and mixed (CFU-MIX) colonies (±SD) are shown of duplicated cultures stimulated with graded volumes of COS/pLB4 CM.

### Induction of AML Proliferation (see Figure 4)

AML blasts were purified using a bovine albumin (BSA) density gradient. Residual T-lymphocytes were removed from the AML samples by E rosette sedimentation (17, 49, 50). AML (patient 1) colony formation was determined not only in the established PHA leukocyte feeder (PHA 1.f) system, but also in a modified version of the technique in which the leukocytes were replaced by COS/pLB4 CM, permitting assessment of its colony-stimulating activity (17, 18, 49, 50) as shown in Figure 4A. All experiments were performed in triplicate. DNA synthesis of AML blasts (patient 2) was assayed by thymidine uptake as described (51) with results shown in Figure 4B. Both assays showed a dose dependent relationship to COS/pLB4 CM added. Addition of control COS medium did not affect AML proliferation in either assay.

### C. Construction of eucaryotic expression vector pLB4/BPV

In order to establish stable cell lines expressing human IL-3, C127 cells (ATCC CRL 1616) were transfected with a derivative of pLB4. This derivative was constructed by insertion of the entire BPV-1 genome (69) into pLB4 by the following strategy. The BPV-1 BamHI fragment was excised from the vector pdBPV-MMTneo(342-12) (70). The BamHI sticky ends were filled in using Klenow polymerase. Then the vector pLB4 was cleaved at the unique EcoRV site within the Eco gpt gene. Subsequently, the blunt-ended BPV-1 fragment was cloned into the EcoRV cleaved pLB4, resulting in the vector pLB4/BPV which is able to replicate in C127 cells. pLB4/BPV was transfected to C127 cells using the calcium phosphate precipitation method (45). The transfected cells were cultured for 16 days, after which foci were picked from the culture dishes. Several independent cell lines were established. The pLB4/BPV vector appears to be stably maintained within the cells, as judged by Southern blotting of Hirt extracts (71) of several cell lines. Conditioned culture medium was tested for IL-3 activity using the AML proliferation assay. The stable cell lines produce active human IL-3.

### Example 3

### Construction of E. coli Expression Vectors

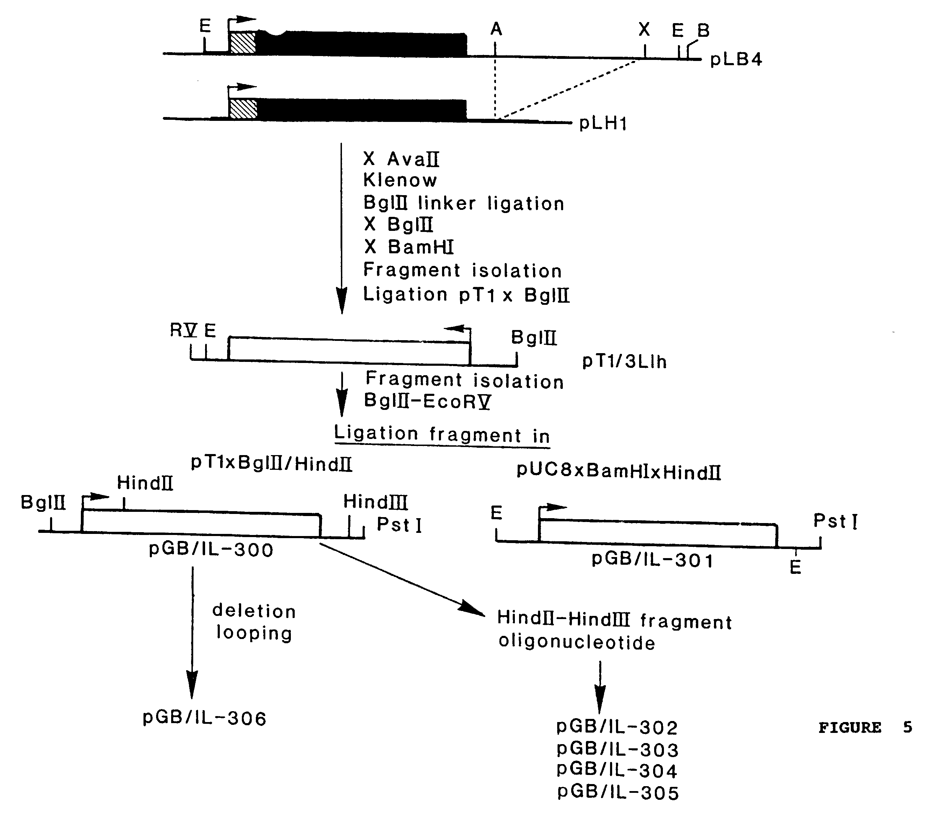

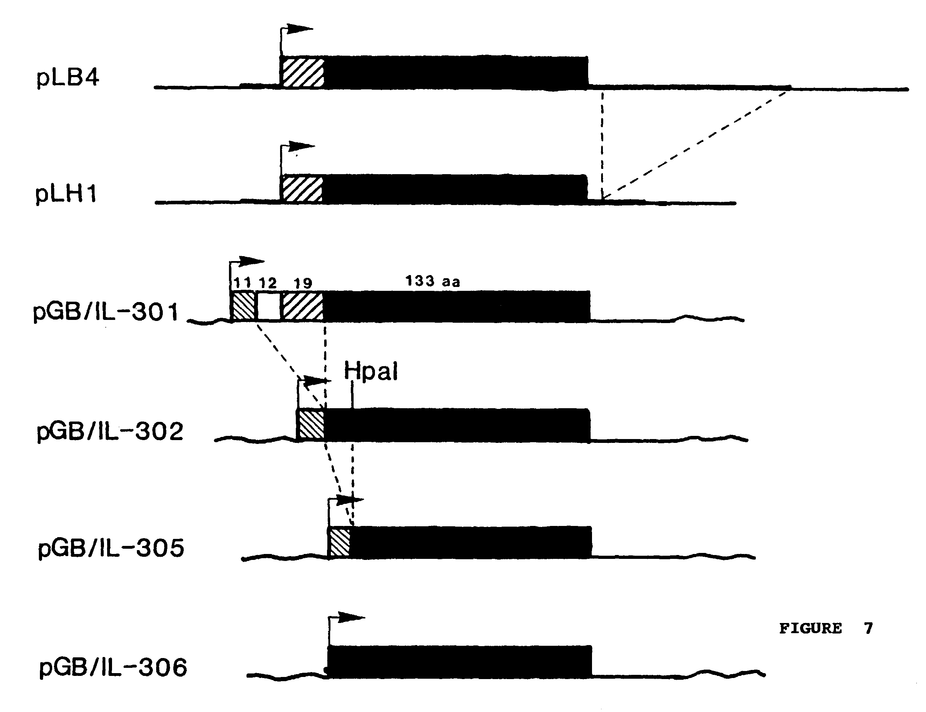

### A. Construction of pGB/IL-301 (see Figures 5, 6, 7 and 8)

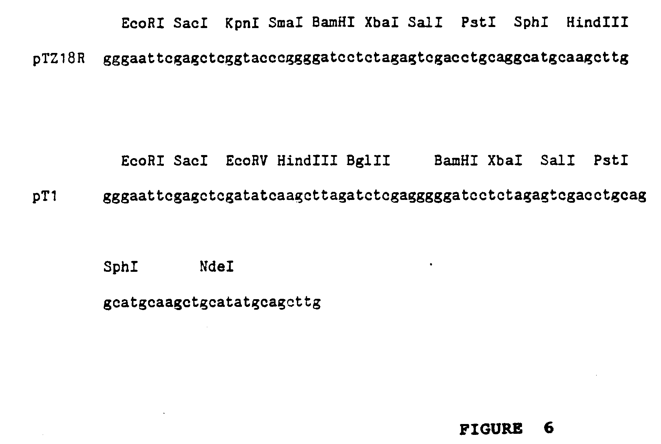

For construction of E. coli expression vectors, the following modifications were performed according to standard procedures (36).
1. The 3'-terminal noncoding sequences between the AvaI site (position 541) and the XhoI site (position 856) in pLB4 were deleted by fusion of the DNA fragments following filling of the sticky ends with Klenow enzyme (Figure 5).
2. For introduction of the hmulti-CSF insert into a bacterial expression vector, the following steps were performed. The pLH1 vector was digested with AvaII and the recessed ends filled with Klenow polymerase. Following ligation of the BglII linker (CAGATCTG), the DNA was digested with BglII and BamHI. The BglII-BamHI hmulti-CSF fragment was purified on polyacrylamide gel and subcloned in the BglII site of pT1, a derivate of pTZ18R (Pharmacia) modified in the multiple cloning site (see Figure 6). Two clones were obtained, which had the insert in the opposite orientation with respect to the lacZ promoter (see Figure 5). Inserts of these two clones were isolated on polyacrylamide gel following digestion with BglII and EcoRV and subcloned in pT1 digested with BglII and HindII. The junction of the BglII linker and the hmulti-CSF DNA was verified by sequence analysis and showed a fusion of the linker to the AvaII site located at nt 1 of the cDNA clone (this AvaII site had arisen by ligation of the EcoRI linker to the cDNA molecule). Since this construct (pGB/IL-300) was not in phase with the lacZ protein, the BglII-EcoRV insert was subcloned into BamHI and HindII digested pUC8 (52). The resulting construct (pGB/IL-301, see Figures 5, 7 and 8) was tested for production of a lacZ/hmulti-CSF fusion protein.

### B. Construction of pGB/IL-302, pGB/IL-303, pGB/IL-304 and pGB/IL-305 (Figures 5, 7 and 8)

Several base changes were introduced into the coding sequence for the N-terminal part of the fusion proteins by introduction of synthetic oligo nucleotides into pGB/IL-300. The new expression vectors, called pGB/IL-302, pGB/IL-303 and pGB/IL-304 were constructed as follows: the HindII-HindIII fragment of pGB/IL-300 was isolated on agarose gel and ligated to a synthetic oligonucleotides comprising the nucleotides 99-137 of hmulti-CSF and a 5' terminal SalI recognition sequence and inserted into pTZ18R digested with SalI and HindIII. The sequence of several clones was established. Indeed, several base changes were observed, resulting in modifications of the hmulti-CSF protein. Inserts of several clones were transferred to pUC8 for expression of the lacZ fusion protein (pGB/IL-302, pGB/IL-303). Clone pGB/IL-304 was made in fase with lacZ by ligation of the SalI site following filling of recessed ends with Klenow. Construction was verified by PvuI digestion. Several clones lacked a synthetic oligonucleotide and were found to be fused in frame to the lacZ protein. One example of these clones was called pGB/IL-305.

### C. Construction of pGB/IL-306 (see Figures 5, 7 and 8)

An expression vector coding for a protein lacking the lacZ N-terminal amino acids was made from pGB/IL-300 by deletion looping as described in (53). The synthetic oligonucleotide comprised 22 nucleotides upstream of the pTZ lacZ gene including the ATG start codon and the first 24 nucleotides coding for mature IL-3. This plasmid was called pGB/IL-306 (Figures 5, 7 and 8).

E. coli strains containing the plasmids pGB/IL-300, pGB/IL-301 and pGB/IL-302 were deposited with CBS on July 13, 1987 under CBS 377.87, CBS 379.87 and CBS 378.87, respectively.

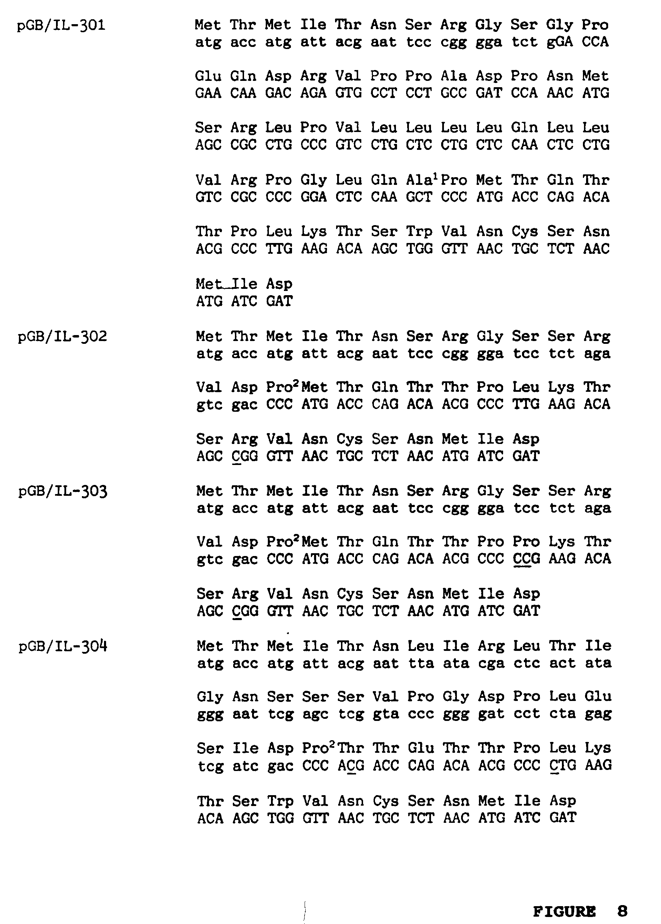

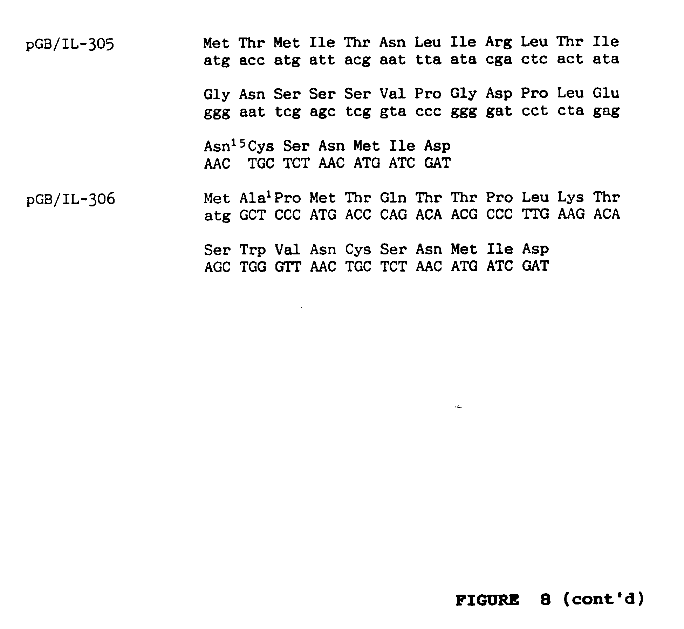

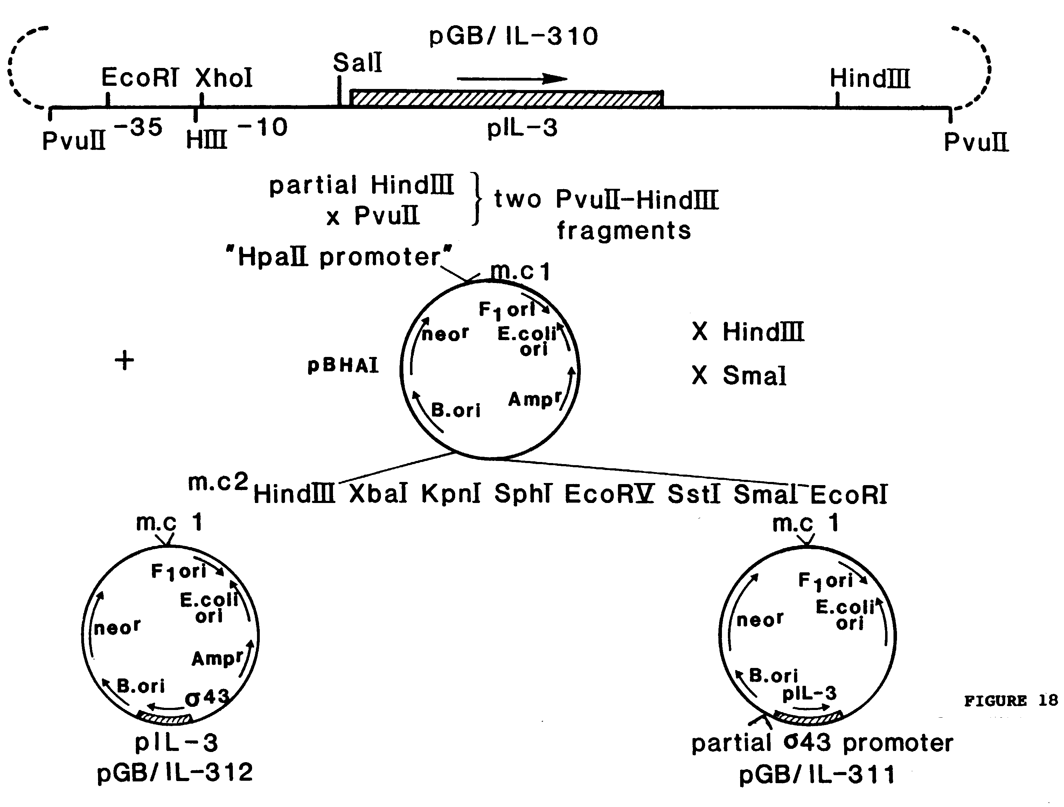

Figure 8 shows the sequence of fusion regions for the various plasmids constructed. The sequence of the clones is given from the start of the lacZ protein coding region in either pUC8 or pTZ18R (lower case letters) and of the hmulti-CSF coding region (upper case letters) up to the ClaI site at position 158. Mutations in the hmulti-CSF DNA sequence are underlined, resulting in trp¹³→arg¹³ (pGB/IL-302); leu⁹→ pro⁹ and trp¹³→arg¹³ (pGB/IL-303); met³→thr³ and a silent change (pGB/IL-304).

In the priority application EP 87201322.2, filed on July 13, 1987, other designations were used for these plasmids as follows:
pGB/IL-300 = pT-hIL3;
pGB/IL-301 = pUC/hmulti;
pGB/IL-302 = pUC/hmultiΔ1A;
pGB/IL-303 = pUC/hmultiΔ1B;
pGB/IL-304 = pUC/hmultiΔ1C;
pGB/IL-305 = pUC/hmultiΔ2;
pGB/IL-306 = pTZ/hmulti;

### D. Expression of lacZ/hmulti-CSF Fusion Proteins and Mature hmulti-CSF in E. coli

E. coli strains (JM 109) carrying various expression vectors were grown in LB medium containing 50 µg/ml of ampicillin at 37°C until an optical density of 0.5 at 550 nm was reached. Subsequently IPTG (isopropyl beta-D-thiogalactoside, Pharmacia) was added to the culture to a final concentration of 1 mM and incubation was continued for 3-4 hours.

Plasmids pGB/IL-306 and pGB/IL-302 were also transformed to E. coli DH1 (wild type lacZ operon). Those strains were grown in LB medium or 2 x TY medium containing 50 µg/ml of ampicillin at 37°C for 16 hours.

Bacteria were collected by centrifugation and sonicated in buffer containing 0.1 M Tris/HCl, pH 8.0; 5mM EDTA 0.2% Nonidet P40 (NP-40) and 1 mM phenylmethylsulfonyl fluoride (PMSF) and centrifuged for 30 min at 20,000 x g. Polyacrylamide gel electrophoresis of the pellet and supernatant fractions showed that the bulk of the hmulti-CSF proteins is stored in the bacteria in an insoluble form.

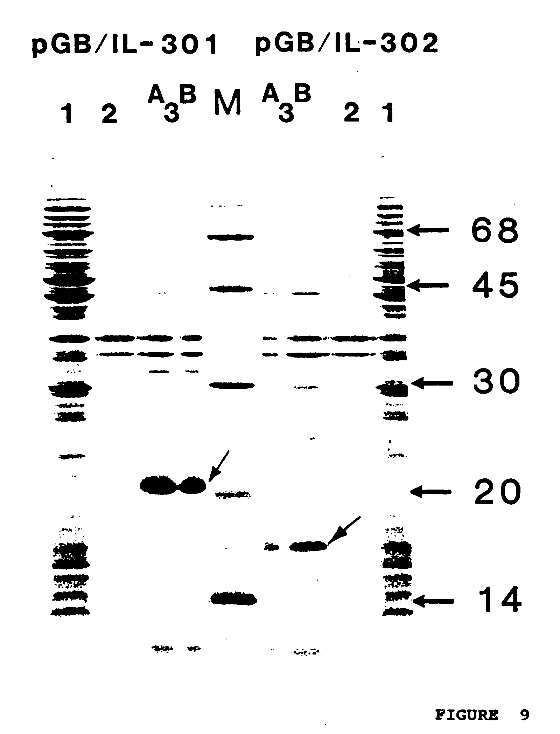

The pellet was re-extracted with 0.5% NP-40 buffer and finally solubilized with 8 M urea 0.1 M Tris/HCl, pH 8.0 and 5mM dithiothreitol. Thus, an extensive purification of the fusion proteins was achieved (Figure 9).

As shown in the figure, inclusion bodies from bacteria (E. coli) containing pGB/IL-301 and pGB/IL-302 were isolated as described. Lanes 1 show the 0.2% NP40 supernatant (sample corresponds to 0.1 ml of the original bacterial culture). Lanes 2 show the 0.5% NP40 supernatant (0.2 ml) and lanes 3 the pellet solubilized in 8M urea buffer (A: 0.05 ml; B: 0.2 ml). The proteins were separated on a 13.5% SDS-polyacrylamide gel and stained with Coomassie Brilliant Blue. Molecular weights (in kDA) of marker proteins (lane M) are denoted on the right. The human multi-CSF fusion proteins are indicated by arrows. The fusion protein encoded by pGB/IL-301 has a MW as expected of about 20 kDA; that produced from pGB/IL-302, of about 16 kDA.

### E. Determination of Biological Activity of Bacterial hmulti-CSF Preparations.

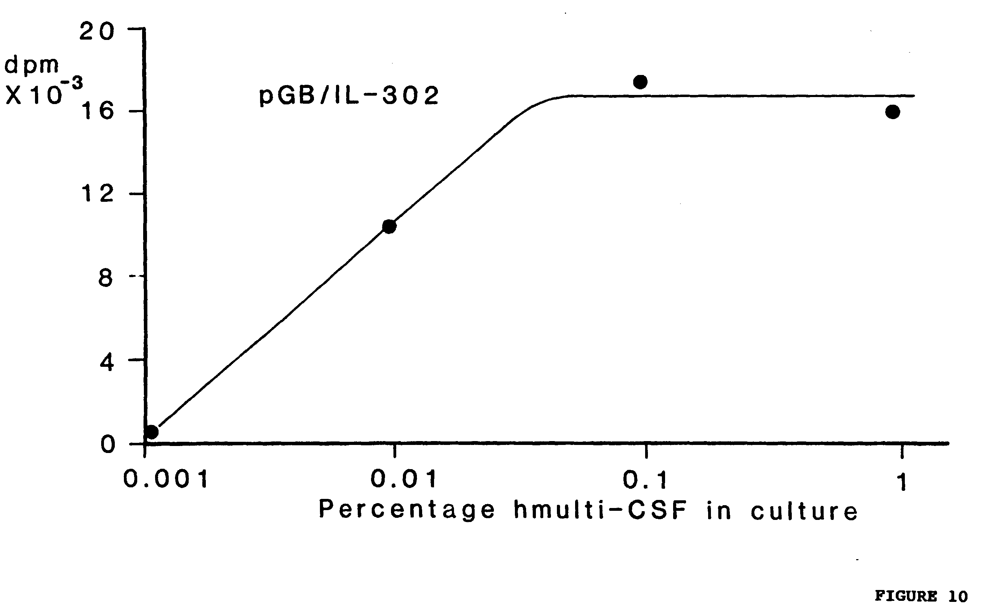

Bacterial protein preparations were diluted in alpha medium containing 1% bovine serum albumin, filter sterilized and assayed in the AML blast proliferation assay. Diluted samples were added to purified AML blasts and cultured for four days. DNA synthesis was measured using ³H thymidine as described (51). One unit per ml is defined as the amount of hmulti-CSF required for half maximal proliferation of AML blasts. Figure 10 shows this titration. Various dilutions of the urea extracted protein preparation of bacteria containing the plasmid pGB/IL-302, were assayed for the stimulation of AML blast proliferation using ³H-thymidine. The fusion protein concentration of this protein preparation was 33 µg/ml. Based on the presented titration curve, the activity of this preparation is 16,000 units/ml.

The amount of bacterial fusion protein in the preparations was estimated from polyacrylamide gel-electrophoresis and used for calculating specific activities.

The results are shown in the following table:

**Table 1**

| Biological Activity of Bacterial hmulti-CSF Preparations | | | | |
|---|---|---|---|---|
| | Mr (x 10⁻³) lacZ/hmulti (1) | ug protein per ml (2) | units per ml (3) | Specific activity units per mg IL-3 |
| pGB/IL-301 | 20 | 20 | 45 | 4,500 |
| pGB/IL-302/303 | 16 | 5 | 2400 | 480,000 |
| pGB/IL-304 | 18 | ND(4) | 18 | - |
| pGB/IL-305 | 16 | 1 | 300 | 300,000 |
| pGB/IL-306 | 15 | ND | 70 | ND |
| 1. Approximate molecular weights are estimated from the DNA sequence of the fusion protein (Figure 8). 2. IL-3 concentrations were estimated on SDS-polyacrylamide gel and calculated per ml of starting culture. 3. Activity of urea solubilized protein was determined in the AML proliferation assay and is expressed per ml of starting culture. 4. Not determined. | | | | |

From these results it was concluded that human multi-CSF expressed as a fusion protein in E. coli was obtained in biologically active form. The results show that changes introduced into the N-terminus of the fusion proteins may influence the specific activity of these proteins.

### Example 4

### Preparation of Antibody Preparations Capable of Immunospecific Reaction with Human IL-3 Protein

### A. Polyclonal Rabbit Anti-Human IL-3 Antiserum.

A preparative gel was made from a lysate of E. coli co taining the plasmid pGB/IL-301. The 20 kDA band with the IL-3 fusion protein was sliced out, minced in saline with a mortar and emulsified in a 1:1 ratio in Complete Freund's Adjuvant containing 1 mg of Mycobacterium tuberculosis H37RA per ml. New Zealand White rabbits (spf) were immunized with 1 ml of the emulsion (with ± 100 µg IL-3 fusion protein) divided over 5 injection sites (2 x i.m. in the thighs, 3 x s.c. on the back). Booster injections of the same antigen in Incomplete Freund's Adjuvant were given at week 2, 4 and 6. Serum was collected at week 8 by venapuncture from the ear.

One volume of serum was absorbed with 9 volumes of sonicated pUC8 containing E. coli (overnight at 4°C) to remove nonspecific antibodies. Immunoblotting of all IL-3 constructs made in E. coli, B. licheniformis, B. subtilis, S. cerevisiae and K. lactis showed immunospecific reaction with the absorbed sera at a dilution of 1 in 6500.

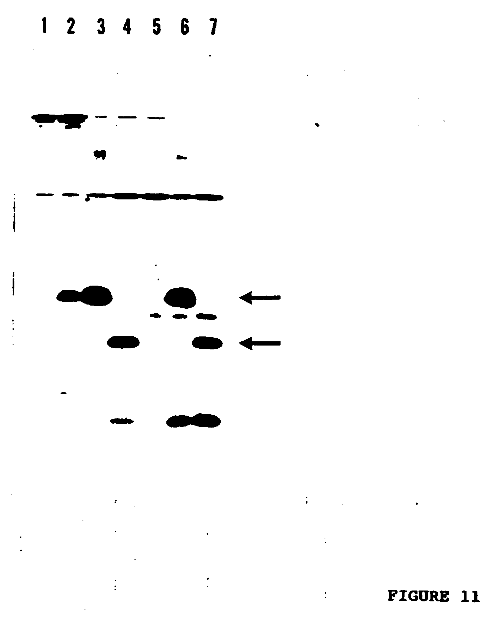

Some of these results are shown in Figure 11. The proteins were isolated from the recombinant hosts as described above and were separated on a 13.5% polyacrylamide gel and blotted onto a nitrocellulose membrane. Lane 1: E. coli containing pTZ18R (control); Lane 2: pGB/IL-301; Lane 3: pGB/IL-301; Lane 4: pGB/IL-302; Lane 5: pUC19 (control); Lane 6: pGB/IL-301; Lane 7: pGB-IL-302. Lanes 6 and 7 show proteins present in the pellet after the sonification of the bacteria. Lanes 3, 4 and 5 show proteins present in the pellet after the first washing step. Lanes 1 and 2 show the final urea-solubilized protein fractions.

The arrows show the fusion proteins (of the expected size) expressed from pGB/IL-301 and pGB/IL-302.

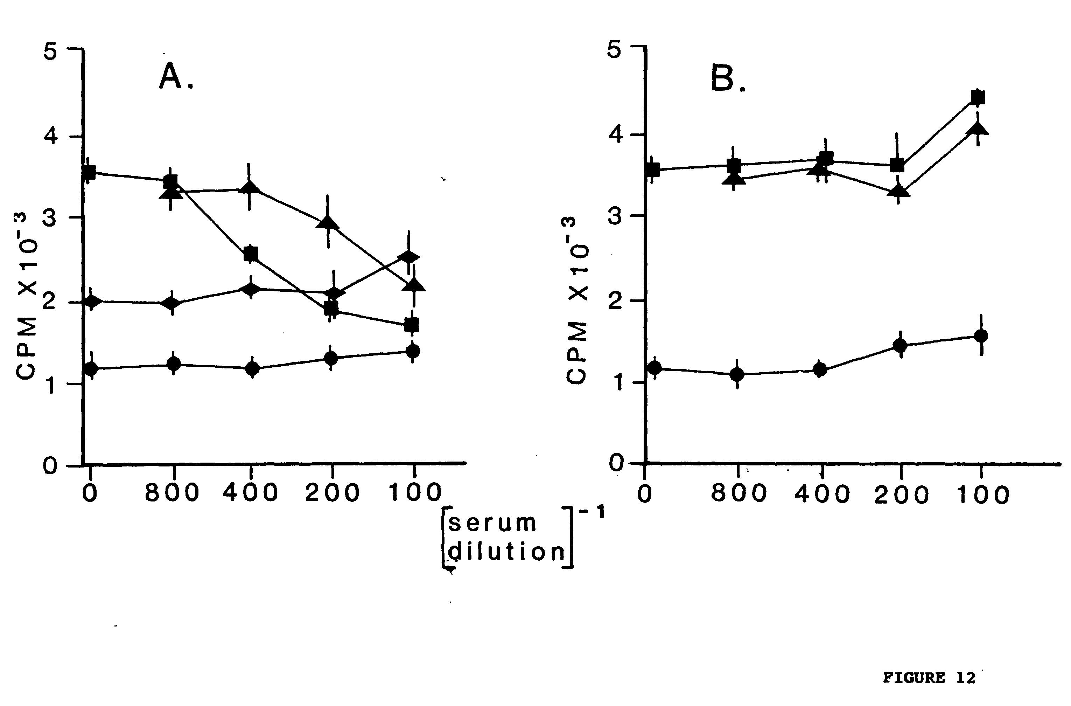

Figure 12A shows the inhibition of IL-3 dependent proliferation of AML blast cells by anti-IL-3 antiserum. Figure 12B shows that the pre-immune serum does not affect the action of IL-3 on AML blast cell proliferation. In both panels, ▲ = IL-3 at 10 U/ml; ■ = IL-3 at 1U/ml; ● = control, no addition.

Figure 12A shows IL-3 dependent growth in the AML blast proliferant assay (51) was inhibited by the sera in a dose dependent manner; Figure 12B shows pre-immune sera do not have this effect. As control, GM-CSF dependent growth was unaffected by these sera in the same assay (Figure 12A where ◆ = GM-CSF at 100 U/ml.)

### B. Monoclonal Mouse Anti-Human IL-3 Antibodies

Balb/C mice were immunized with 3 x 0.1 ml (s.c.) of the same emulsion as used for the rabbits. A booster (0.1 ml i.p.) of antigen in Incomplete Freund's Adjuvant was given at week 2 and three days later spleen lymphocytes were fused with SP2/0 myeloma cells according to standard procedures (65). Hybridoma supernates were screened in the Enzyme Linked Immunosorbent Assay, using a lysate of E. coli pGB/IL-302 (containing the 17 kDA IL-3 fusion product) as a positive control and a lysate of E. coli pUC8 as negative control. In total, 29 IL-3 hybridoma cultures secreting antibodies specific for IL-3 were selected and stabilized.

### Example 5

### Construction of Bacillus expression vectors

General cloning techniques were used (36).

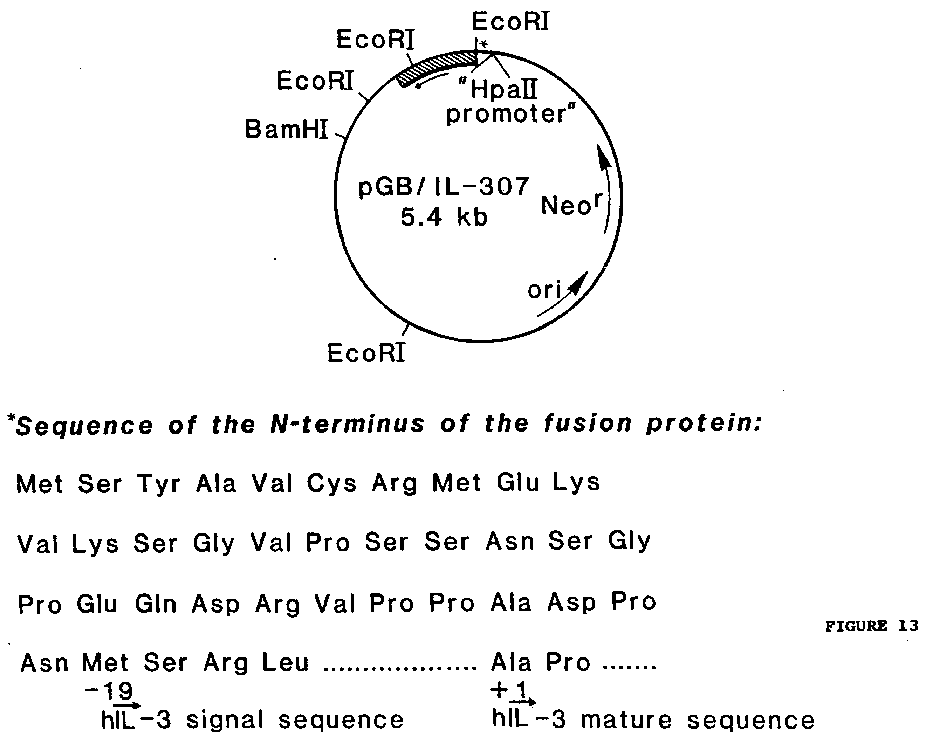

### A. Construction of pGB/IL-307 (Figure 13)

For construction of pGB/IL-307 the SmaI fragment of pLB4 carrying the hmulti-CSF gene, was ligated into PvuII digested pUB110 (54). After transformation to competent cells (56) of DB105 (a spo- derivative of the protease deficient strain DB104 (55)), two clones were obtained, as expected: the fragment was cloned in both orientations. The plasmid that harbored the fragment in the correct orientation with respect to the so-called "Hpa II promoter" (57) was called pGB/IL-307. In this case a fusion protein will be made (see Figure 13).

### B. Construction of pGB/IL-310

A hmulti-CSF expression plasmid was prepared as described below.

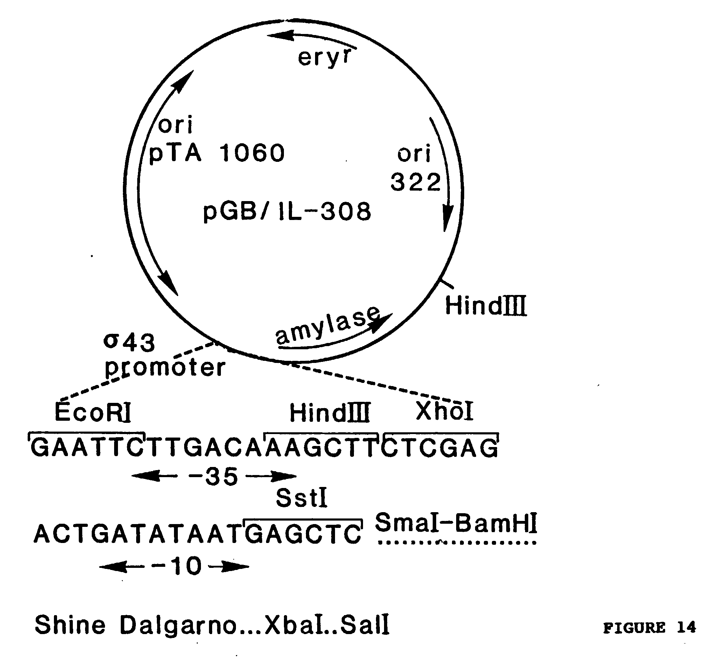

### 1. Promoter cloning (Figure 14).

For expression in Bacillus a synthetic σ⁴³ promoter as described (58) is used (the promoter used to be called σ⁵⁵).

Plasmids pPROM55s (58), the promoter containing plasmid, and pGPA14 (59) were digested with EcoRI and XbaI. The promoter fragment was ligated into the vector fragment, which had been purified on an agarose gel. After transformation to E. coli (JM 101), the correct plasmid was obtained and called pGB/IL-308 (Fig. 14).

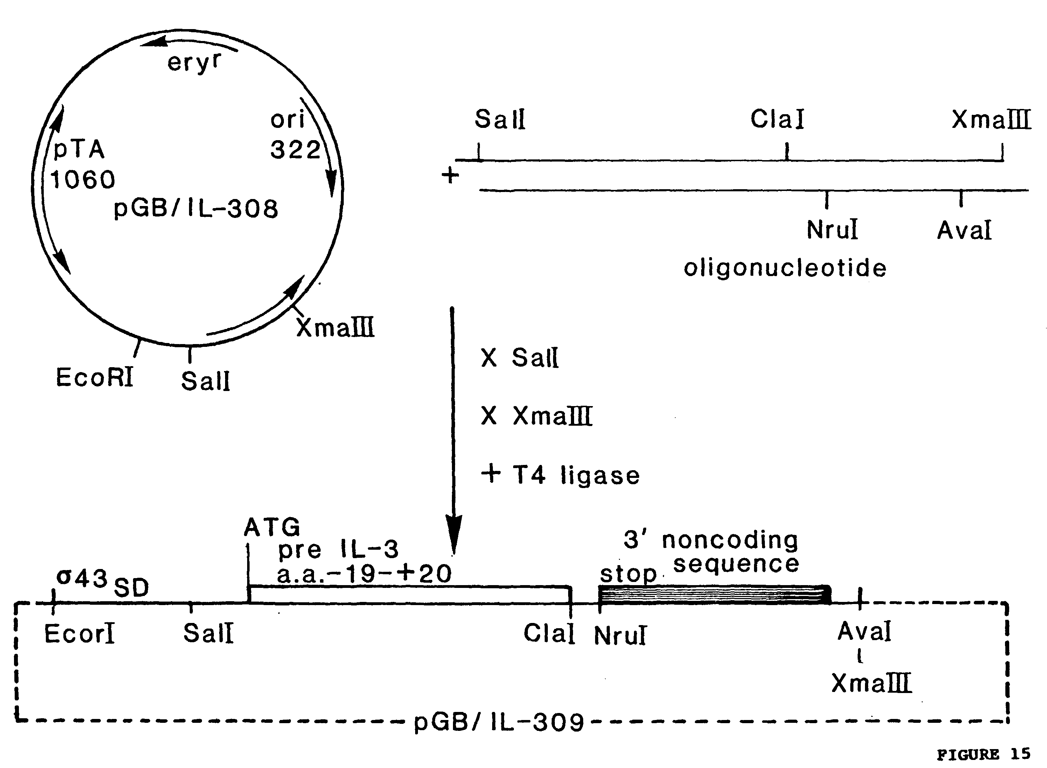

### 2. Introduction of a synthetic oligonucleotide into pGB/IL-308 (Figure 15).

A synthetic oligonucleotide comprising the nucleotides 39-158 and 484-546 of hmulti-CSF, a 5' terminal SalI recognition sequence and a 3' terminal XmaIII site was ligated into SalI-XmaIII digested pGB/IL-308. The ligation mixture was introduced into JM101. After analysis of a number of transformants, the correct plasmid was found, pGB/IL-309.

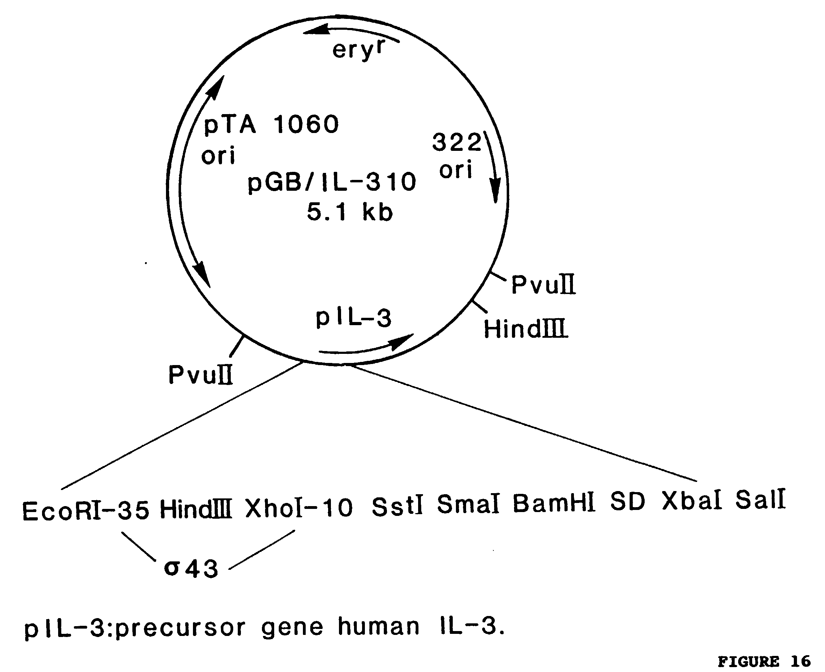

### 3. Introduction of hIL3 (Figure 16).

After transformation to and isolation from B. subtilis DB105, the plasmid pGB/IL-309 was digested with XmaIII. The recessed ends were filled in with Klenow polymerase, and the plasmid was cleaved with ClaI. The plasmid pGB/IL-307 was digested with AvaI, the ends filled in with Klenow and then digested with ClaI. Subsequently, the hmulti-CSF containing fragment was ligated into the pGB/IL-309 fragment and transformed to JM101. The resulting plasmid was called pGB/IL-310 (Figure 16). This plasmid harbored the hIL-3 gene with its own signal sequence. After isolation of the correct plasmid, it was also introduced into B. subtilis DB105.

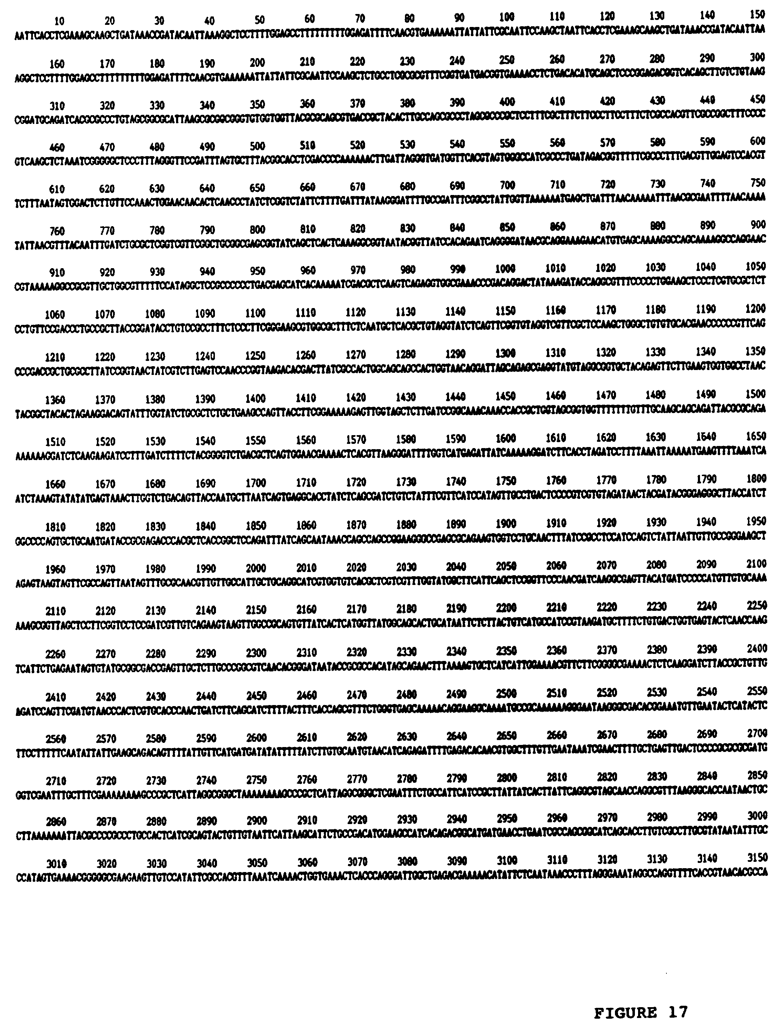

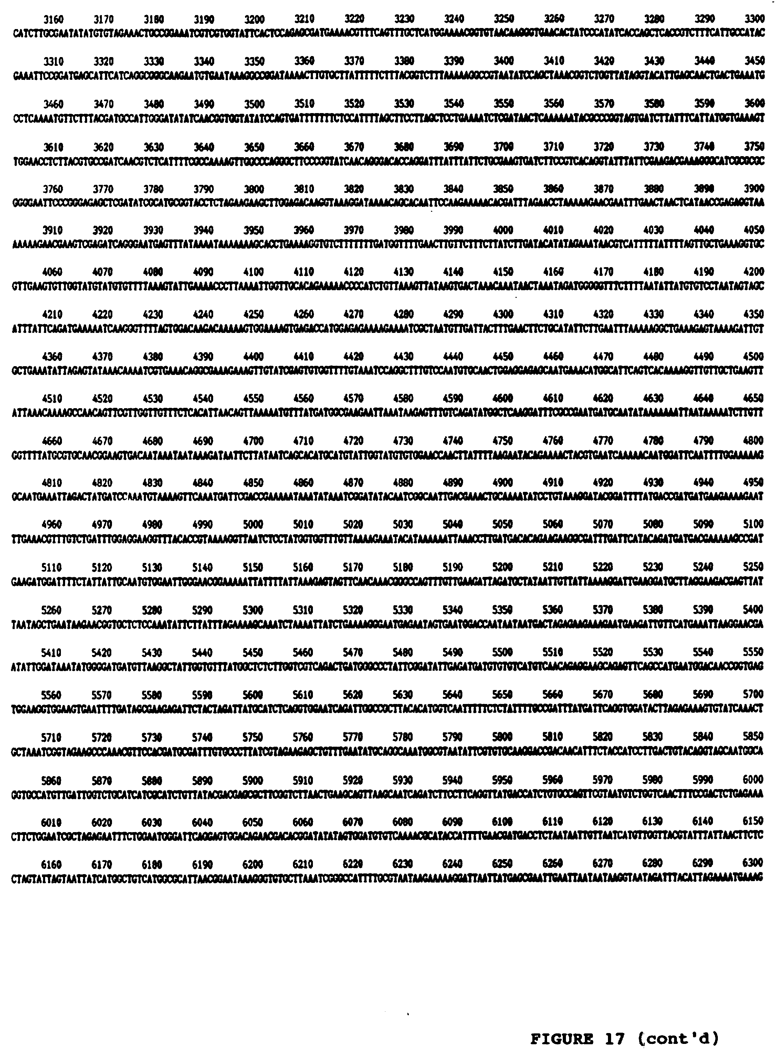

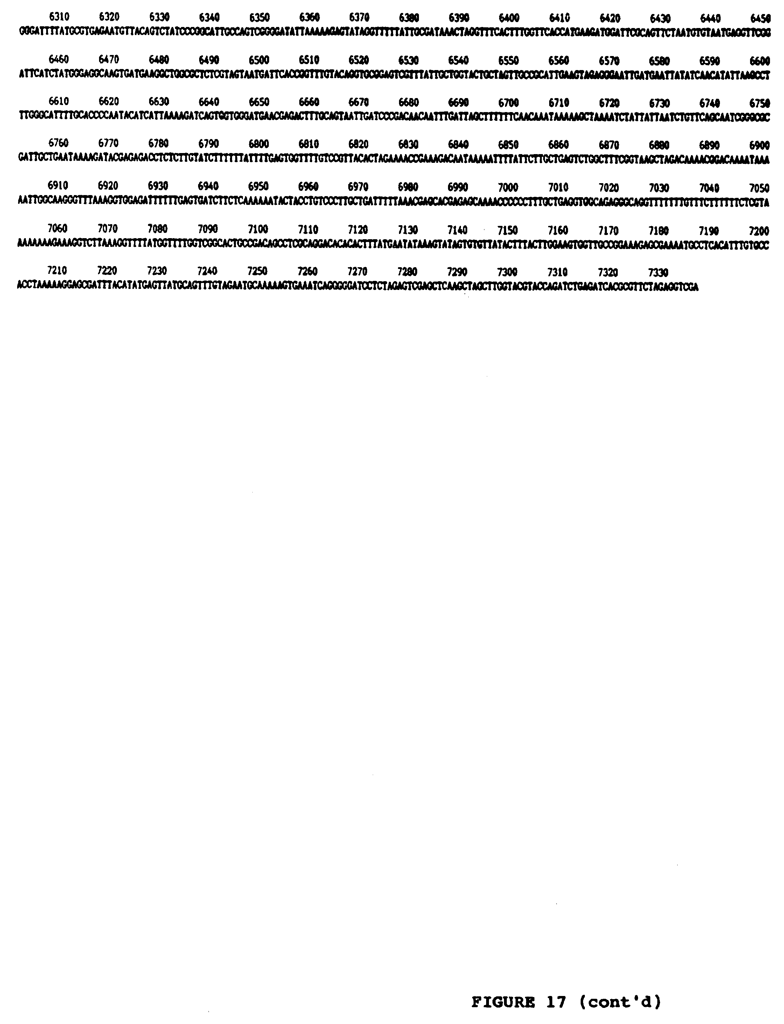

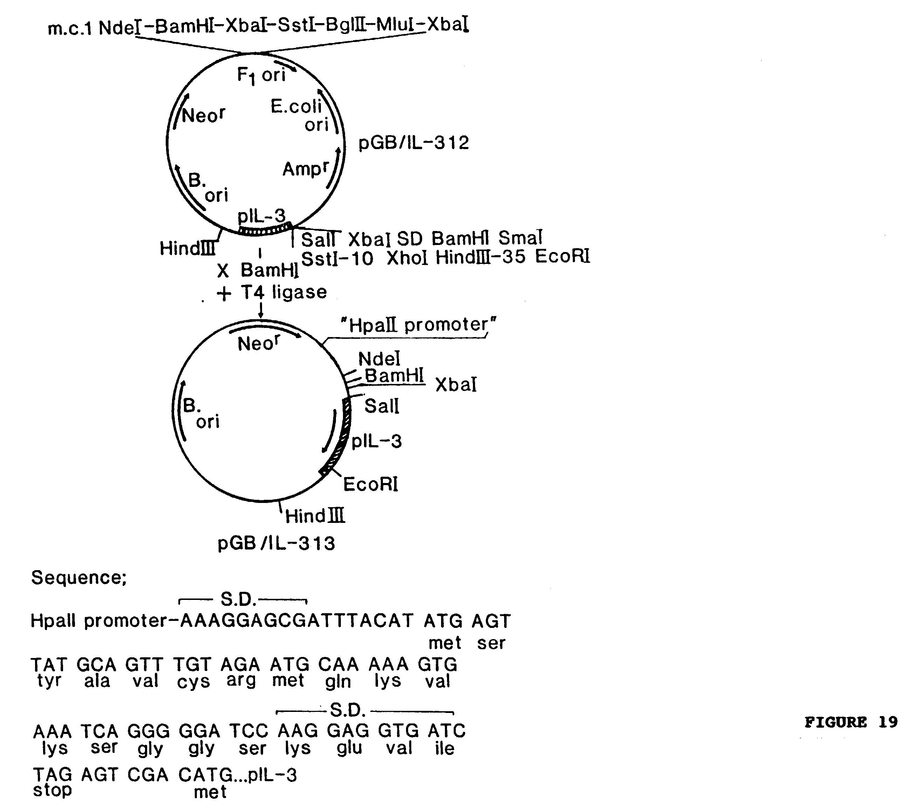

### C. Construction of pGB/IL-311 and pGB/IL-312 (Figures 17, 18)

pGB/IL-310 was partially digested with HindIII and totally with PvuII. The two hmulti-CSF containing PvuII-digested with HindIII and SmaI.

Figure 17 shows the nucleotide sequence of plasmid pBHA1. The plasmid consists of positions 11-105 and 121-215; bacteriophage FD terminator (double): positions 221-307; a part of plasmid pBR322 (viz. positions 2069-2153): positions 313-768; bacteriophage Fl, origin of replication (viz. positions 5482-5943): positions 772-2571; part of plasmid pBR322, viz. the origin of replication and the beta-lactamase gene: positions 2572-2685; transposon Tn903, complete genome: positions 2719-2772; tryptophan terminator (double): positions 2773-3729; transposon Tn9, the chloramphenicolacetyltransferase gene. The nucleotides at position 3005 (A), 3038 (C), 3302 (A) and 3409 (A) differ from the wild type cat coding sequence. These mutations were introduced so as to eliminate the NcoI, Ball, EcoRI and PvuII sites: positions 3730-3804; multiple cloning site: positions 3807-7264; part of plasmid pUBllO, viz. the replication function and kanamycin resistance gene (EcoRI-PvuII fragment) (66, 67): positions 7267-7331; multiple cloning site. The fragments were put together by known cloning techniques, e.g. filling in of sticky ends with Klenow, adapter cloning, etc. All data were derived from Genbank^{R}, National Nucleic Acid Sequence Data Bank, NIH, USA.

After transformation to JM101 and analysis of a number of ampicillin resistant colonies, two different plasmids were found: pGB/IL-312, which harbored the complete gene with complete control sequences, and pGB/IL-311, which contained the complete gene and the promoter lacking the -35 region in the other orientation (see Figure 18).

pGB/IL-311 has been transformed to B. subtilis DB105 and B. licheniformis strain T399 (Δamy, spo⁻, exoprotease negative, rif^{r}, see ref. 68, where this strain is T9).

### D. Construction of pGB/IL-313 (Figure 19).

In order to obtain a smaller plasmid, with the hmulti-CSF gene behind the "HpaII promoter", pGB/IL-312 was digested with BamHI and religated. The ligaton mixture was transformed into DB105 competent cells. A number of neomycin resistant colonies were analysed and the correct plasmid was obtained. The plasmid was called pGB/IL-313.

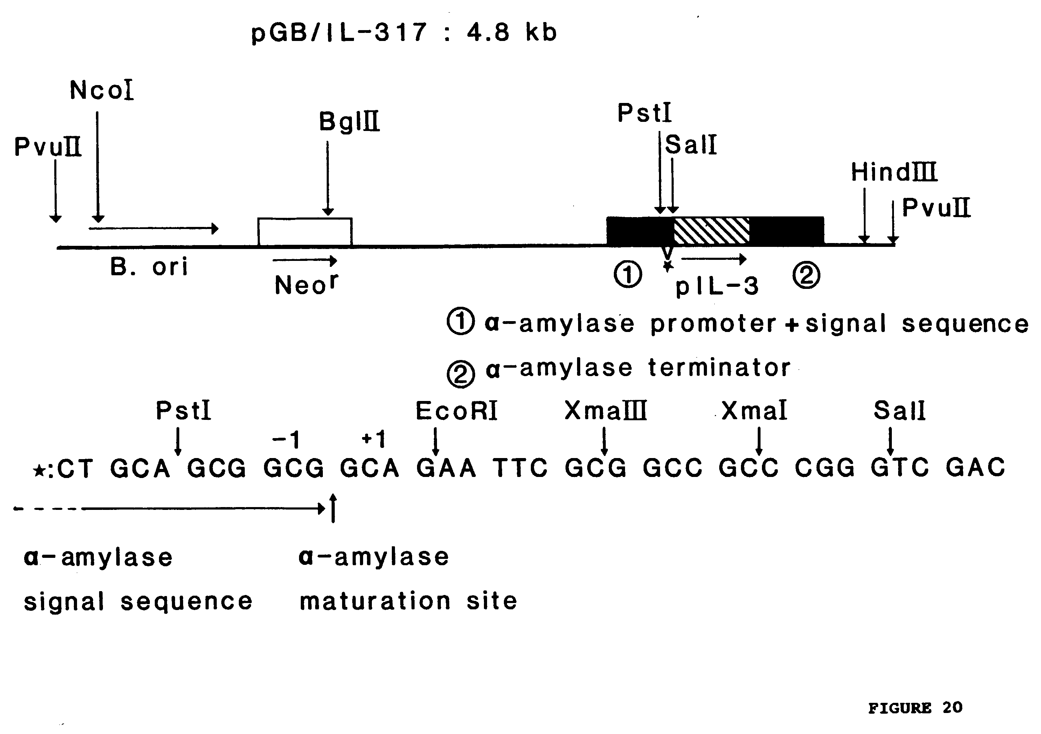

### E. Construction of pGB/IL-317 (Figure 20)

In order to clone the hmulti-CSF gene behind the B. licheniformis alpha-amylase transcriptional and translational initiation region and signal sequence, one of the earlier described pOL5-delta vectors (68) was used, viz. pOL5-2 delta. Besides the alpha-amylase signal sequence (29 amino acids long) this plasmid harbors one amino acid of the alpha-amylase mature sequence (an Ala) followed by a multiple cloning site: EcoRI-XmaIII-XmaI-SalI-HindIII (68).

The SalI-PvuII fragment of plasmid pGB/IL-310 containing the hmulti-CSF gene was ligated into the SalI-PvuII digested pOL5-2 delta vector and transformed to DB105. The resulting plasmid was called pGB/IL-317 (Figure 20). The hIL-3 gene still harbors its own signal sequence on this plasmid. The plasmid was also introduced into B. licheniformis T399.

### F. Expression of Five Expression Plasmids in Bacillus Strains

B. subtilis and B. licheniformis strains carrying the expression plasmids mentioned below were grown in TSB medium containing 20 µg/ml neomycin or 10 µg/ml erythromycin at 37°C (for 16-24 hours); 300 µg/ml of the culture was centrifuged. The pellet was resuspended in sample buffer and analyzed using polyacrylamide gel-electrophoresis followed by Western blotting. The supernatant was TCA precipitated, and the pellet was resuspended in sample buffer. Both supernatant and pellet were analyzed for IL-3 protein (see Table 2).

To determine the biological activity of the produced proteins, the following steps were carried out: The cellpellets were resuspended in a buffer containing 0.1 M Tris/HCl pH 8.0 and 10 mM MgCl₂. Lysozyme was added to a final concentration of 1 mg/ml and PMSF to a final concentration of 1 mM. The solution was incubated for 30 min. at 37°C. Subsequently DNase (final concentration 20 µg/ml) was added and the solution was incubated for 15 min. at 20°C. Finally, the biological activity of this preparation as well as of the supernatant of the cultured cells was determined as described. The results are shown in Table 2.

**Table 2**

| Expression of the Bacillus Vectors | | | | | |
|---|---|---|---|---|---|
| Plasmid | Strain | MW IL-3 | | Biological | |
| | | Pellet | supernatant | activity | |
| | | (kDA) | (kDA) | pellet | supernatant |
| pGB/IL-307 | DB105 | 21 | - | + | - |
| pGB/IL-310 | DB105 | 15;17 | 15;17 | - | - |
| pGB/IL-311 | DB105 | 12.5;15 | - | + | - |
| | T399 | - | - | + | - |
| pGB/IL-313 | DB105 | 15;17 | 12.5;15 | + | - |
| | T399 | - | - | + | - |
| pGB/IL-317 | DB105 | 12.5;15 | 12.5;15 | + | + |
| | | 17;20 | 17 | | |
| | T399 | 12.5;15 | 12.5;15 | + | + |
| | | 17;20 | 17 | | |

It can be concluded, that in B. subtilis, using pGB/IL-307, a fusion protein is made that has IL-3 activity. When the human IL-3 gene only contains its own signal sequence no significant secretion of human IL-3 is obtained. All IL-3 activity is found intracellularly. In those cases it seems that besides precursor IL-3 mature IL-3 (15 kd) has been formed in the cell. Thus, some transport across the membrane might have taken place, but the protein is not transported across the cell wall. However, using the alpha-amylase regulation and secretion signals (pGB/IL-317) most of the IL-3 activity appeared to be secreted into the culture medium. Besides a degradation product, two proteins are detected in the supernatant, one of about 15 kDA and one of about 17 kDA, most probably mature IL-3 and precursor IL-3, respectively. These data indicate that both processing sites, viz. the alpha-amylase and the hmulti-CSF processing site, are used. In the cell the most abundant product is precursor IL-3 containing the alpha-amylase signal sequence (the 20 kDA protein) as shown by Western blotting. Sometimes a degradation product is detected.

### Example 6

### Construction of Kluyveromyces lactis expression vectors

### A. Construction of pGB/IL-316

A DNA fragment comprising the Tn5 gene (61) conferring resistance to gentamycin G418, under the direction of the alcohol dehydrogenase I (ADHI) promoter from S. cerevisiae, similar to that described by Bennetzen and Hall (62), was inserted into the SmaI site of pUC19 (63). An E. coli strain containing the obtained plasmid, pUC-G418, was deposited with CBS on December 4, 1987 under CBS 872.87.

Into the XbaI-HindIII cleaved pUC-G418 vector a XbaI-HindIII fragment from plasmid pGB903 (64) containing the K. lactis lactase promoter and calf prochymosin DNA was inserted, resulting in plasmid pGB/IL-314.

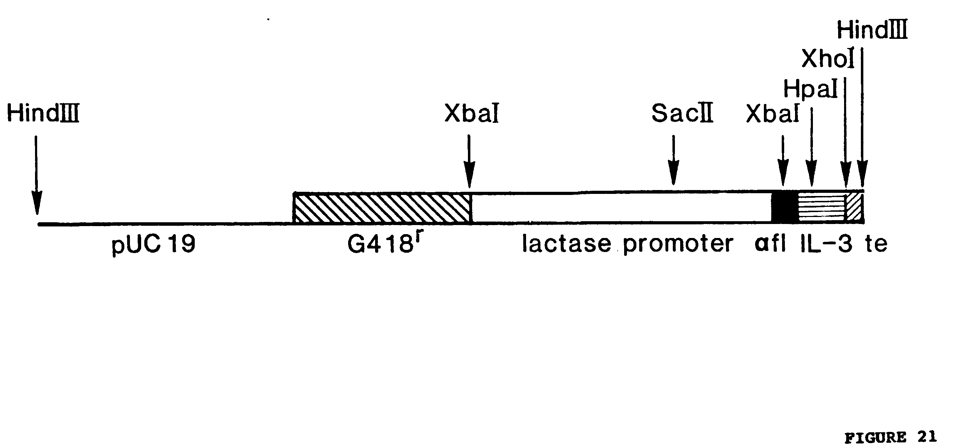

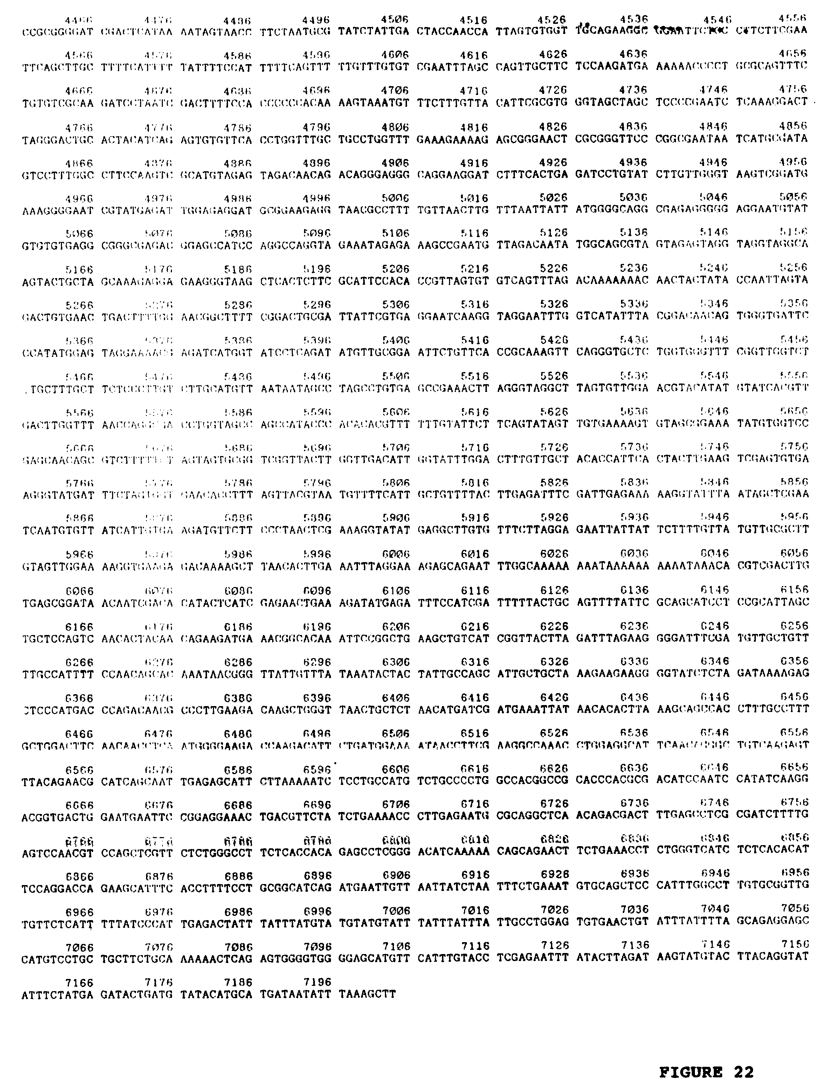

The SalI-HindIII fragment from this plasmid was replaced by a synthetic DNA fragment containing a small multiple cloning site and the lactase terminator (see Figures 21, 22). The resulting plasmid is designated pGB/IL-315.

In the SacII-XhoI cleaved pGB/IL-315 vector the following fragments were ligated:
1. The SacII-XbaI fragment from pKS105 (U.S. Pat. Appln. Ser. 078,539, 64), carrying the 3' part of the lactase promoter and the 5' part of the alpha-factor signal sequence of S. cerevisiae.
2. A synthetic oligonucleotide comprising the 3' part of the alpha-factor signal sequence starting at the XbaI site and the 5' part of the mature hIL-3 cDNA sequence up to the 5' half of the HpaI site (aa-residue 14).
3. The HpaI-XhoI fragment carrying most part of the hIL-3 cDNA sequence (residue 15-133 plus the 3' non-coding region). The resulting plasmid, designated pGB/IL-316, is depicted schematically in Figure 21. The complete vector sequence from the SacII site in the lactase promoter sequence up to the HindIII site at the end of the synthetic terminator is given in Figure 22.

Figure 22 shows the nucleotide sequence of plasmid pGB/IL-316 between the unique SacII site in the lactase promoter and the Hind III site behind the terminator (residues 4457 to 7204). Residues 4457 to 6100 comprise the lactase promotor sequence. Residues 6101 to 6355 comprise the alpha factor signal sequence. Residues 6356 to 7115 comprise the sequence for mature human IL-3 plus the 3' noncoding cDNA sequence. Residues 7116 to 7204 comprise the synthetic terminator sequence.

### B. Construction of pGB/IL-318

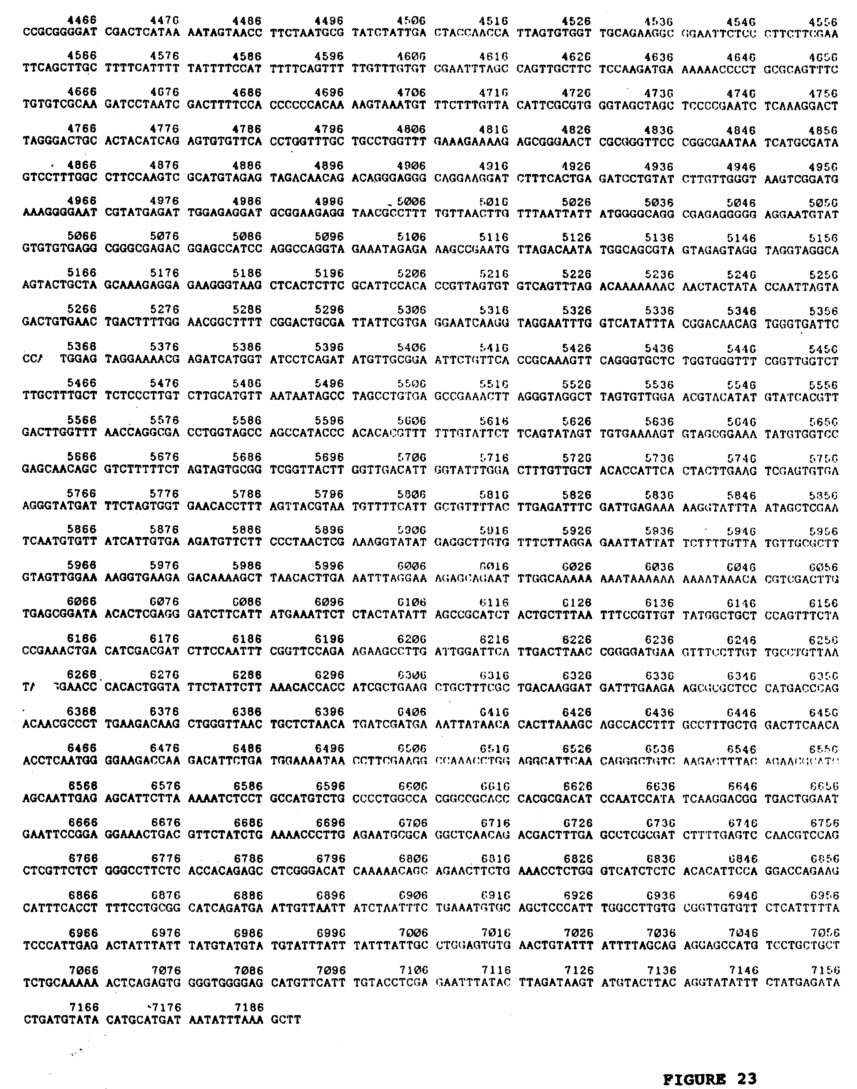

An expression vector similar to pGB/IL-316 was constructed in which the coding information for the alpha factor signal sequence of S. cerevisiae was replaced by the alpha-factor signal sequence of K. lactis (64). The remaining part of the plasmid is identical to pCB/IL-316. The sequence of pGB/IL-318 between the SacII site in the lactase promoter and the HindIII site behind the terminator (residues 4457 to 7190) is given in Figure 23.

Residues 4457 to 6087 comprise the sequence of the lactase promoter and a small linker sequence. Residues 6088 to 6342 comprise the K. lactis alpha factor signal sequence. Residues 6343 to 7102 comprise the sequence for mature human IL-3 plus the 3' noncoding cDNA sequence. Residues 7103 to 7190 comprise the synthetic terminator sequence.

### C. Transformation of Kluyveromyces Lactis and Analysis of Secreted hIL-3

Plasmids pGB/IL-316 and pGB/IL-318 were digested at the unique SacII site in the lactase promoter region, and used to transform K. lactis strain CBS 2360 (see 64). Integration of the plasmids is thus targeted to the chromosomal lactase gene promoter region. The resulting G418 resistant transformants were grown to saturation in liquid YEPD medium, and the culture supernatants and cell lysates were assayed for IL-3 activity using the AML cell DNA synthesis assay.

Virtually all IL-3 appeared to be secreted into the culture medium, and to be active. The proteins from the culture supernatant were precipitated using ethanol and analyzed using denaturing polyacrylamide gel-electrophoresis followed by Western blotting. The predominant product has an apparent MW of about 21 kDA, whereas also a distinct band at about 15 kDA is observed. The latter product most probably corresponds to the mature unglycosylated IL-3, whereas the 21 kDA product is the product carrying core glycosylation at the two potential glycosylation sites. Incubation with Endoglycosidase H results in a protein migrating in the 15 kDA range, suggesting that all IL-3 is processed correctly during the secretion process and that the bulk of the protein is being glycosylated.

### Example 7

### Construction of a Saccharomyces Cerevisiae Expression Vector

### A. Construction of pGB/IL-319

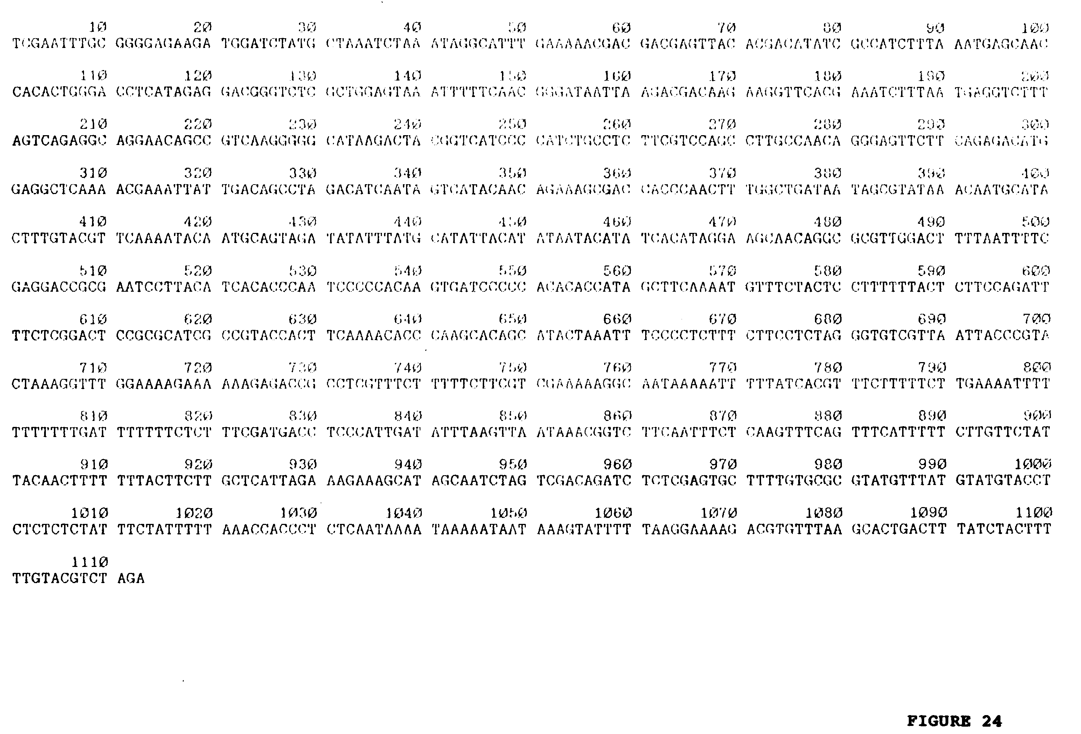

First an expression vector called pGB/TEFact was constructed. On this pTZ18R (Pharmacia) derived plasmid the S. cerevisiae translation elongation factor (EF-lalpha) promoter sequence, which was cloned and sequenced as described (73,74), is coupled by means of a small SalI-BglII-XhoI linker to the S. cerevisiae actin transcription terminator sequence (75), which was synthesized using an Applied Biosystems DNA synthesizer. The sequence of the expression cassette is given in Figure 24. Residues 1 to 949 comprise the EF-lalpha promoter. Residues 950 to 967 comprise the sequence of the SalI-BglII-XhoI linker. Residues 968 to 1113 comprise the actin terminator sequence.

The unique SmaI site in pGB/TEFact was used to introduce the G418 resistance cassette described in Example 6. The resulting plasmid was called pGB/TEFactG418.

Finally, the hIL-3 expression vector pGB/IL-318 was constructed by introduction of the following DNA sequences into the SalI-XhoI cleaved pGB/TEFactG418 plasmid:
- The SalI-NruI fragment from pGB/IL-316 carrying the S. cerevisiae alpha factor signal sequence and the hIL-3 coding sequence upto the NruI site.
- A synthetic NruI-XhoI DNA fragment comprising the remaining nucleotides coding for hIL-3 and the XhoI recognition sequence immediately following the TGA stopcodon.

### B. Transformation of Saccharomyces Cerevisiae and Analysis of Secreted hIL-3

Plasmid pGB/IL-319 was cleaved at the unique EcoRI site in the EF-1α promoter. Integration of the plasmid is thus targeted to the chromosomal EF-1α region. S. cerevisiae wild type strain D273-103 (alpha; ATCC 25657) was transformed as described for K. lactis (64). The G418-resistant colonies were picked and transformants were given to saturation in liquid YEPD medium. The culture supernatant was assayed for hIL-3 activity using the AML assay. The protein produced by S. cerevisiae was found biologically active.

The proteins from the supernatant were precipitated using ethanol and subsequently analyzed by polyacrylamide gel-electrophoresis followed by Western blotting. Two prominent products could be distinguished on the Western blot, a 21 kDA glycosylated product and an unglycolysed product of about 15 kDA.

### REFERENCES

1 Metcalf D., Blood 67, 257-267 (1986).
2 Whetton A.D. and Dexter T.M. TIBS 11, 207-211 (1986).
3 Wagemaker G., In "Bone Marrow Transplantation" " (eds. Van Bekkum D.W. and Lowenberg B.) Marcel Dekker Inc. New York 1-72 (1985).
4 Dorssers L. et. al., Exp. Hematol. 12, 357, 1984.
5 Till J.E. and McCulloch E.A., Radiat. Res. 14, 213-222 (1961).
6 Hapel A.J., et. al., Blood 65, 1453-1459 (1985).
7 Scheven B.A.A., Nature 321, 79-81 (1986).
8 Garland J.M. and Crompton S. Exp. Hematol. 11, 757-761 (1983).
9 Stanley E.R. et. al., Cell 45, 667-674 (1986).
10 Kreigler A.B. et. al., Blood 60, 503-508 (1982).
11 Fung M.C. et. al., Nature 307, 233-237 (1984).
12 Yokata T. et. al., Proc. Natl. Acad. Sci. USA, 81, 1070-1074 (1984).
13 Lowenberg B. and Dicke K.A., Exp. Hematol. 5, 319-331 (1977).
14 Wagemaker G. and Peters M.F., Cell. Tiss. Kinet. 11, 45-56 (1978).
15 Ihle J.N., et. al., In "advances in viral oncology", vol. 4 (ed Klein G.) 95-137, Raven Press, New York 1984.
16 Fauser A.A. and Messner H.A. Blood 52, 1243-1248 (1978).
17 Lowenberg B. et. al., Leuk. Res. 4, 143-149 (1980).
18 Lowenberg B. et. al., Blood 59, 64-645 (1982).
19 Buick R.N. et. al., Blood 54, 95-104 (1979).
20 Huynh T.V. et. al., In "DNA cloning", vol. 1 (Ed. Glover D.M.) IRL press, Oxford 45-78 (1985).
21 Kozak M. Cell 44, 283-292 (1986).
22 Von Heijne G. Eur J. Biochem 133, 17-21 (1983).
23 Perlman D. and Halvorson H.O., J. Mol. Biol. 167, 391-409 (1983).
24 Shaw G. and Kamen R. Cell 46, 659-667 (1986).
25 Schrader J.W., et. al., Proc. Natl. Acad. Sci. USA 83, 2458-2462 (1986).
26 March C.J., et. al., Nature 315, 641-647 (1985).
27 Higashi Y. et. al., J. Biol. Chem. 258, 9522-9529 (1983).
28 Dijkema R. et. al., EMBO J. 4, 761-767 (1985).
29 Zwarthoff E.C. et. al., Nucleic Acid Res. 13, 791-804 (1985).
30 Clark-Lewis I. et. al., Science 231, 134-139 (1986).
31 Kindler V. et. al., Proc. Natl. Acad. Sci. USA 83, 1001-1005 (1986).
32 DeLamarter J.F. et. al., EMBO J. 10, 2575-2581 (1985).
33 Lemischka I.R. et. al., Cell 45, 917-927 (1986).
34 Yu-Chung Yang et. al., Cell 47, 3-10 (1986).
35 Miyatake S. et. al., Proc. Natl. Acad. Sci. USA 82, 316-320 (1985).
36 Maniatis T. et. al., In "Molecular Cloning, A. laboratory manual". Cold Spring Harbor Laboratories, New York (1982).
37 Gubler U. and Hofmann B.J., Gene 25, 263-269 (1983).
38 Feinberg A.P. and Vogelstein B. Anal. Biochem. 132, 6-13 (1983).
39 Sanger F. et. al., Proc. Natl. Acad. Sci. USA 74, 5463 (1977).
40 Queen C. and Korn L.J. Nucleic Acid Res. 12, 581-599 (1984).
41 Staden R. Nucleic Acid Res. 10, 2951-2961 (1982).
42 Devereux J., et. al., Nucleic Acid Res. 12, 387-395 (1984).
43 Lipman D.J. and Pearson W.R. Science 227, 1435-1441 (1985).
44 Subramani S. and Southern P.J., Anal. Bioch. 135, 1-15 (1983).
45 Wigler M., et. al., Cell 14, 725-731 (1978).
46 Majdic O., et. al., Int. J. Cancer 33, 617-623 (1984).
47 Lowenberg B. and Bauman J.G.J. Blood 66, 1225-1232 (1984).
48 Delwel R., et. al., Blood 68, 41-45 (1986)
49 Swart K. et. al., Blood 59, 816-821 (1982).
50 Swart K. and Lowenberg B. Cancer Res. 44, 657-660 (1984).
51 Touw I. et. al., Blood 68, 1088-1094 (1986).
52 Vieira, J. and Messing J., Gene 19, 259-268 (1982)
53 Osinga, K.A. et al., Nucleic Acids Res. 11, 8595-8608 (1983)
54. Gryczan, T.C. et al., J. Bacteriology 134, 318-329 (1978)
55. Kawamura, F. and Doi, R.H., J. Bacteriology 160, 442-444 (1984)
56. Bron, S. and Venema, G., Mutat. Res. 15, 1-10 (1972).
57. Zyprian, E. and Matzura, H., DNA 5, 219-225 (1986).
58. EPA 0224294, published June 3, 1987.
59. EPA 0244042, published November 4, 1987.
60. Stanssens P. et al., In "Protein Engineering and Site-Directed Mutagenesis". Twenty-Fourth Harden Conference. Program and Abstracts (1985) (Fersht, A.R. and Winter, G., edts).
61. Reiss, B. et al., EMBO J. 3, 3317-3322 (1984).
62. Bennetzen, J.L. and Hall, B.D., J. Biol. Chem. 257, 3018-3025 (1982).
63. Yanisch-Perron, C. et al., Gene 33, 103-119 (1985).
64. U.S. Appl. Ser. No. 078,539, filed July 28, 1987.
65. Salfre, S. and Milstein, C., Meth Enz 73, 3-75 (1981).
66. McKenzie, T. et al., Plasmid 15, 93-103 (1986).
67. McKenzie, T. et al., Plasmid 17, 83-85 (1987).
68. European Patent Application 87201379.2, filed July 20, 1987.
69. Chen, E.Y. et al., Nature 299, 529-534 (1982).
70. Law, M-F. et al., Mol. Cell Biol. 3, 2110-2115 (1983)
71. Hirt, B., J. Mol. Biol. 26, 365-367 (1967).
72. Suarez Rendueles, M.P. et al., FEBS Lett. 131, 296-300 (1981).
73. Najata, S. et al., EMBO J. 3, 1825-1830 (1984).
74. Nagashima, K. et al., Gene 45, 265-273 (1986).
75. Gallwitz, D. and Sures, I., Proc. Natl. Acad. Sci. USA 77, 2546-2550 (1980).

## Claims

1. A transformed living host cell containing genetic material derived from recombinant DNA-material and coding for human IL-3 having a Pro at position 8 of the mature protein molecule and having nucleotide sequence "H" of Figure 1.

2. A transformed living host cell according to Claim 1, which is selected from the group consisting of yeasts, bacteria, fungi and tissue culture cells.

3. A transformed yeast host cell according to Claim 2, which is selected from the group of Saccharomyces and Kluyveromyces.

4. A transformed bacterium host cell according to Claim 2, which is selected from the group of E. coli and Bacillus.

5. A transformed tissue culture host cell according to Claim 2, which is selected from the group of COS cells, and C127 cells having the deposit accession number ATCC CRL 1616.

6. An expression system operable in a recombinant host which expression system consists of the DNA sequence "H" of Figure 1 encoding human IL-3 having a Pro at position 8 of the mature protein molecule, operably linked to control sequences effective in said host.

7. The expression system of Claim 6, wherein the DNA encoding human IL-3 contains no introns.

8. The expression system of Claim 6 or 7, wherein the control sequence comprises a promoter selected from the group consisting of the lac promoter, the HpaII promoter, the δ43 promoter, the alpha-amylase promoter, the EF-1 alpha promoter and the SV40 promoter.

9. A recombinant host cell according to any one of Claims 1 to 5, transformed with the expression system of any one of Claims 6 to 8.

10. A recombinant DNA sequence containing no introns which encodes human IL-3 having Pro at position 8 of the mature protein molecule and which further comprises the nucleotide sequence shown as encoding amino acids 1-133 in sequence "H" of Figure 1.

11. A method for producing human IL-3 having an amino acid sequence corresponding with nucleotide sequence "H" of Figure 1 by a microbial host cell, said method comprising:
introducing into said host cell a DNA construct comprising an expression cassette which comprises in the direction of transcription a transcriptional initiation regulatory region functional in said host cell;
a DNA sequence encoding human IL-3 with the nucleotide sequence "H" of Figure 1; and
a transcriptional termination regulatory region functional in said host cell;
growing said host cell comprising said DNA construct in a nutrient medium under suitable culture conditions, whereby human IL-3 is produced; and
recovering the human IL-3 product.

12. A method according to claim 11, wherein said host cell is defined in any one of Claims 1 to 5 or 9.

13. A purified protein having human IL-3 activity and an amino acid sequence corresponding with nucleotide sequence "H" of Figure 1, substantially free of other substances accompanying said protein.

14. The protein of Claim 13, which is produced by the recombinant expression of the DNA sequence shown as encoding amino acids 1-133 in sequence "H" of Figure 1.

15. The protein of Claim 13 or 14, in glycosylated or unglycosylated form.

16. The protein of any one of Claim 13 to 15, produced in a transformed host cell as defined in any one of Claim 1 to 5 or 9.

## Patentansprüche

1. Transformierte lebende Wirtszelle, die genetisches Material enthält, das von rekombinantem DNA-Material abgeleitet ist und für menschliches IL-3 kodiert, das ein Pro auf Position 8 des Reife-Protein-Moleküls und eine Nukleotidsequenz ,,H" nach Figur 1 hat.

2. Transformierte lebende Wirtszelle nach Anspruch 1, die aus der Gruppe ausgewählt ist, die aus Hefen, Bakterien, Pilzen und Gewebekulturzellen besteht.

3. Transformierte lebende Hefewirtszelle nach Anspruch 2, die aus der Gruppe der Saccharomyces und der Kluyveromyces ausgewählt ist.

4. Transformierte Bakterienwirtszelle nach Anspruch 2, die aus der Gruppe der E. Coli und der Bazillen ausgewahlt ist.

5. Transformierte Gewebekulturwirtszelle nach Anspruch 2, die aus der Gruppe der COS-Zellen ausgewählt ist und der C127-Zellen, die die Hinterlegungsnummer ATCC CRL 1616 haben.

6. Ein in einem rekombinanten Wirt betreibbares Expressionssystem, wobei das Expressionssystem die DNA-Sequenz _{''}H" nach Figur 1 enthält, die menschliches IL-3 verschlüsselt, das ein Pro auf Position 8 des Reife-Protein-Moleküls hat, und die operativ mit Kontrollsequenzen verbunden ist, die in besagtem Wirt wirksam sind.

7. Expressionssystem nach Anspruch 6, wobei das DNA-verschlüsselnde menschliche IL-3 kein Intron enthält

8. Expressionssystem nach Anspruch 6 oder 7, wobei die Kontrollsequenz einen Promotor enthält, der aus der Gruppe ausgewählt ist, die aus dem lac-Promotor, dem Hpall-Promotor, dem δ43-Promotor, dem Alpha-Amylase-Promotor, dem EF-1-Alpha-Promotor und dem SV40-Promotor besteht.

9. Rekombinante Wirtszelle nach einem der Ansprüche 1 bis 5 transformiert mit dem Expressionssystem nach einem der Ansprüche 6 bis 8.

10. Recombinante DNA-Sequenz, die kein Intron enthält und die menschliches IL-3 verschlüsselt, das ein Pro auf Position 8 des Reife-Protein-Moleküls hat und die weiterhin die Nukleotidsequenz enthält, die als verschlüsselnde Aminosäuren 1 - 133 in Sequenz _{''}H" nach Figur 1 dargestellt ist.

11. Verfahren zum Produzieren von menschlichem IL-3, das eine Aminosäuresequenz aufweist, die mit der Nukleotidsequenz ,,H" nach Figur 1 korrespondiert, durch eine mikrobische Wirtszelle, wobei besagtes Verfahren enthält:
das Einführen eines DNA-Konstrukts in besagte Wirtszelle, das eine Expressionskassette enthält, die in der Transkriptionsrichtung eine regulative Region zur Transkriptionsinitialisierung aufweist, die in besagter Wirtszelle funktional ist;
eine DNA-Sequenz, die menschliches IL-3 mit der Nukleotidsequenz ,,H" nach Figur 1 verschlüsselt; und
eine regulative Region zur Transkriptionsbeendung, die in besagter Wirtszelle funktional ist;
ein Anwachsen besagter Wirtszelle, die besagtes DNA-Konstrukt enthält, in einem Nährstoffmedium unter angemessenen Kulturbedingungen, wobei menschliches IL-3 produziert wird; und
Zurückgewinnen des menschlichen IL-3-Produktes.

12. Verfahren nach Anspruch 11, wobei besagte Wirtszelle durch einen der Ansprüche 1 bis 5 oder 9 definiert ist.

13. Gereinigtes Protein, das menschliche IL-3-Aktivität aufweist und eine Aminosäuresequenz, die mit der Nukleotidsequenz ,,H". nach Figur 1 korrespondiert, und im wesentlichen frei ist von anderen Substanzen, die besagtes Protein begleiten.

14. Protein nach Anspruch 13, das durch die rekombinante Expression der DNA-Sequenz produziert wird, die als verschlüsselnde Aminosäuren 1 - 133 in Sequenz _{''}H" nach Figur 1 dargestellt ist.

15. Protein nach Anspruch 13 oder 14 in glykosylierter oder unglykosylierter Form.

16. Protein nach einem der Ansprüche 13 bis 15 produziert in einer transformierten Wirtszelle nach einem der Ansprüche 1 bis 5 oder 9.

## Revendications

1. Cellule hôte vivante transformée, contenant du matériel génétique provenant de matériel d'ADN recombiné et codant l'IL-3 humaine ayant un résidu Pro à la position 8 de la molécule de protéine mature et ayant la séquence nucléotidique "H" de la figure 1.

2. Cellule hôte vivante transformée selon la revendication 1, laquelle est sélectionnée dans le groupe constitué par les levures, les bactéries, les champignons et les cellules de cultures tissulaires.

3. Cellule hôte vivante transformée selon la revendication 2, laquelle est sélectionnée dans le groupe constitué par Saccharomyces et Kluyveromyces.

4. Cellule hôte bactérienne transformée selon la revendication 2, laquelle est sélectionnée dans le groupe constitué par E.coli et Bacillus.

5. Cellule hôte de culture tissulaire transformée selon la revendication 2, laquelle est sélectionnée dans le groupe des cellules COS et des cellules C127 ayant le numéro de dépôt ATCC CRL 1616.

6. Système d'expression fonctionnant dans un hôte recombiné, lequel système d'expression est constitué par la séquence d'ADN "H" de la figure 1 codant l'IL-3 humaine ayant un résidu Pro à la position 8 de la molécule de protéine mature, liée fonctionnellement à des séquences de contrôle efficaces dans ledit hôte.

7. Système d'expression selon la revendication 6, où l'ADN codant l'IL-3 humaine ne contient pas d'introns.

8. Système d'expression selon la revendication 6 ou 7, où la séquence de contrôle comprend un promoteur sélectionné dans le groupe constitué par le promoteur lac, le promoteur HpaII, le promoteur δ43, le promoteur α-amylase, le promoteur EF-1α et le promoteur SV40.

9. Cellule hôte recombinée selon l'une quelconque des revendications 1 à 5, transformée par le système d'expression selon l'une quelconque des revendications 6 à 8.

10. Séquence d'ADN recombiné ne contenant pas d'introns, laquelle code l'IL-3 humaine ayant un résidu Pro à la position 8 de la molécule de protéine mature et comprend en outre la séquence nucléotidique dont il a été montré qu'elle code les acides aminés 1 à 133 dans la séquence "H" de la figure 1.

11. Procédé de production d'IL-3 humaine ayant une séquence d'acides aminés correspondant à la séquence nucléotidique "H" de la figure 1 par une cellule hôte microbienne, ledit procédé comprenant :
l'introduction dans ladite cellule hôte d'un produit de synthèse d'ADN comprenant une cassette d'expression qui comprend, dans le sens de la transcription, une région régulatrice d'initiation de la transcription, fonctionnelle dans ladite cellule hôte ; une séquence d'ADN codant l'IL-3 humaine avec la séquence nucléotidique "H" de la figure 1 ; et une région régulatrice de terminaison de la transcription, fonctionnelle dans ladite cellule hôte ;
la culture de ladite cellule hôte comprenant ledit produit de synthèse d'ADN dans un milieu nutritif dans des conditions de culture appropriées, de l'IL-3 humaine étant produite ; et
la récupération du produit IL-3 humaine.

12. Procédé selon la revendication 11, où ladite cellule hôte est définie selon l'une quelconque des revendications 1 à 5 ou 9.

13. Protéine purifiée ayant une activité IL-3 humaine et une séquence d'acides aminés correspondant à la séquence nucléotidique "H" de la figure 1, sensiblement exempte d'autres substances accompagnant ladite protéine.

14. Protéine selon la revendication 13, laquelle est produite par l'expression recombinée de la séquence d'ADN dont il a été montré qu'elle code les acides aminés 1 à 133 dans la séquence "H" de la figure 1.

15. Protéine selon la revendication 13 ou 14, sous une forme glycosylée ou non glycosylée.

16. Protéine selon l'une quelconque des revendications 13 à 15, produite dans une cellule hôte transformée selon l'une quelconque des revendications 1 à 5 ou 9.
